# EUROPEAN PATENT SPECIFICATION

(11) **EP 0 093 620 B1**
(45) Date of publication and mention of the grant of the patent: **07.01.1987**
(21) Application number: 83302513.3
(22) Date of filing: 04.05.1983
(51) Int. Cl.: C07C 125/065, C07C 155/02, C07C 143/83, A01N 47/20, A01N 47/24

(54) **Fungicidal N-phenylcarbamates**
N-Phenylcarbamate mit fungizider Wirkung
N-phénylcarbamates fongicides

(30) Priority: 04.05.1982 JP 75769/82; 05.05.1982 GB 8212944
(43) Date of publication of application: 09.11.1983
(73) Proprietor: SUMITOMO CHEMICAL COMPANY, LIMITED, Chuo-ku Osaka 541 (JP)
(72) Inventor: Noguchi, Hiroshi, Toyonaka Osaka (JP); Kato, Toshiro, Takarazuka Hyogo (JP); Takahashi, Junya, Nishinomiya (JP); Ishiguri, Yukio, Takarazuka Hyogo (JP); Yamamoto, Shigeo, Ikeda Osaka (JP); Kamoshita, Katsuzo, Toyono-gun Osaka (JP)
(74) Representative: Allard, Susan Joyce

## Description

This invention relates to fungicidal N-phenylcarbamates.

Benzimidazole and thiophanate fungicides such as Benomyl (methyl 1-(butylcarbamoyl)benzimidazol-2-ylcarbamate, Fuberidazol (2-(2-furyl)benzimidazole), Thiabendazole (2-(4-thiazolyl)benzimidazole), Carbendazim (methyl benzimidazol-2-ylcarbamate), Thiophanate-methyl (1,2-bis(3-methoxycarbonyl-2-thioureido)benzene), Thiophanate (1,2-bis(3-ethoxycarbonyl-2-thioureido)benzene, 2-(O,S-dimethylphosphorylamino)-1-(3'-methoxycarbonyl-2'-thioureido)benzene and 2-(0,0-dimethylthiophosphorylamino)-1-(3'-methoxycarbonyl-2'-thioureido)benzene are known to show an excellent fungicidal activity against various plant pathogenic fungi, and they have been widely used as agricultural fungicides since 1970. However, their continuous application over a long period of time results in phytopathogenic fungi developing a tolerance to them, whereby their plant disease-preventative effect is lowered. Furthermore, the fungi which gain tolerance to certain benzimidazole or thiophanate fungicides also show considerable tolerance to other benzimidazole or thiophanate fungicides. Thus, they are apt to obtain a cross-tolerance. Therefore, if any significant decrease in their plant disease-preventive effect in certain fields is observed, their application in such fields has to be discontinued. However, it is often observed that the density of drug-resistant organisms does not decrease even long after the application is discontinued. Although other types of fungicides have to be employed in these cases, only a few are as effective as benzimidazole and thiophanate fungicides in controlling various phytopathogenic fungi. Cyclic imide fungicides such as Procymidone (3-(3',5'-dichlorophenyl)-1,2-dimethylcyclopropane-1,2-dicarboximide), Iprodione (3-(3' ,5' -dichlorophenyl)-1-isopropylcarbamoylimidazolidine-2,4-dione), Vinclozoline (3-(3',5'-(dichlorophenyl)-5-methyl-5-vinyloxazolidin-2,4-dione), ethyl (RS)-3-(3',5'-dichlorophenyl)-5-methyl-2,4-dioxooxazolidine-5-carboxylate, etc., which are effective against various plant diseases, particularly those caused by Botrytis cinerea, have the same defects as previously explained with respect to the benzimidazole and thiophanate fungicides.

C.R. Acad. Sc. Paris, t. 289, S'erie D, pages 691-693 (1979), discloses that herbicides such as Barban (4-chloro-2-butynyl N-(3-chlorophenyl)carbamate), Chlorobufam (1-methyl-2-propynyl N-(3-chlorophenyl)carbamate), Chlorpropham (isopropyl N-(3-chlorophenyl)carbamate) and Propham (isopropyl N-phenylcarbamate) exhibit a fungicidal activity against certain organisms tolerant to some benzimidazole and thiophanate fungicides. However, their fungicidal activity against the drug-resistant fungi is not strong enough, and hence, in practice they cannot be used as fungicides.

We have now found that certain N-phenylcarbamates show an excellent fungicidal activity against plant pathogenic fungi which have developed a resistance to benzimidazole, thiophanate and/or cyclic imide fungicides.

Accordingly, the present invention provides a fungicidal composition which comprises as active ingredient a fungicidally effective amount of an N-phenylcarbamates of the formula: 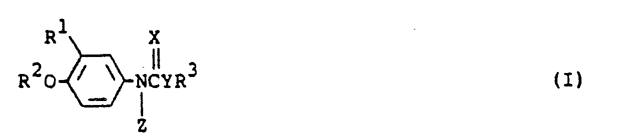 wherein R¹ is a C₂-C₄ alkyl group, a lower alkenyl group, a lower cyanoalkenyl group, a lower alkynyl group, a lower alkyl group substituted with at least one hydroxy or cyano group, or a group of the formula: 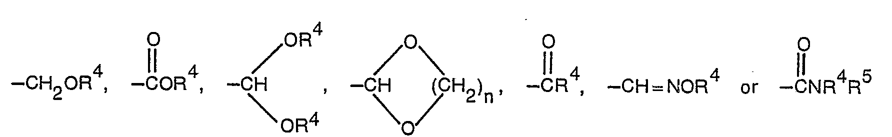 in which R⁴ is a lower alkyl group, a lower alkenyl group, a lower alkynyl group or a lower haloalkyl group, R⁵ is a hydrogen atom or a lower alkyl group and n is 2, 3 or 4; R² is a lower alkyl group, a lower alkenyl group, a lower alkynyl group or a lower alkyl group substituted with at least one halogen atom, lower alkoxy or lower cycloalkyl group; R³ is a C₁-Cₐ alkyl group, a C₃₋C₈ alkenyl group, a C₃-C₈ alkynyl group, a lower cycloalkyl group, a lower haloalkenyl group, a lower haloalkynyl group, a lower aralkyl group or a lower alkyl group substituted with at least one halogen atom, cyano, lower alkoxy, lower alkenyloxy, lower haloalkoxy, phenoxy, lower aralkyloxy or lower cycloalkyl group; X and Y are each independently an oxygen atom or a sulfur atom; and Z is a hydrogen atom, a lower alkyl group, a lower alkenyl group, a lower alkynyl group, a lower cycloalkyl group, a lower haloalkenyl group, a lower alkyl group substituted with at least one halogen atom, cyano, lower alkoxy or lower alkoxycarbonyl group, or a group of the formula: -COR⁶ or -S0₂R⁶ in which R⁶ is a lower alkyl group, a lower alkenyl group, a lower alkynyl group, a lower cycloalkyl group, a lower haloalkenyl group, a lower alkyl group substituted with at least one halogen atom, cyano, lower alkoxy, lower cycloalkyl or phenoxy group (the said phenoxy group being optionally substituted with at least one halogen atom and/or at least one alkyl group), a phenyl group, a furyl group, a thienyl group, a phenyl group substituted with at least one halogen atom, cyano, nitro, trifluoromethyl, lower alkyl or lower alkoxy group, or an aralkyl group (the said aralkyl group being optionally substituted with at least one halogen atom and/or at least one alkyl group), together with an inert carrier or diluent. It is notable that their fungicidal potency against organisms tolerant to benzimidazole, thiophanate and/or cyclic imide fungicides (hereinafter referred to as "drug-resistant fungi" or "drug-resistant strains") is much higher than that against the organisms sensitive to benzimidazole, thiophanate and/or cyclic imide fungicides (hereinafter referred to as "drug-sensitive fungi" or "drug-sensitive strains").

By the term "lower" as used herein in connection with organic radicals or compounds is meant that such radicals or compounds each contain not more than 6 carbon atoms.

Several derivatives of N-(3-trifluoromethyl-4-alkoxyphenyl)carbamate and N-(3-chloro-4-alkoxyphenyl)carbamate which are closely related in structure to the compounds of the present invention have hitherto been synthesized, and are described in the art. For example, isopropyl N-(3-trifluoromethyl-4-methoxyphenyl)carbamate (British Patent 1,021,945), 2-fluoroethyl N-(3-trifluoromethyl-4-methoxyphenyl)carbamate (German Offen. 2,061,678), isopropyl N-(3-chloro-4-propargyloxyphenyl)carbamate (U.S. Patent 3,933,470), methyl N-[3-chloro-4-(2-fluoroethoxy)phenyl]-carbamate (Japan Kokai 77-139721) and methyl N-(3-chloro-4-difluoromethoxyphenyl)thiolcarbamate (EP-A-19759) have been known to exhibit herbicidal or insecticidal activity. Additionally propargyl (3-trifluoromethyl-4-methoxyphenyl)carbamate and propargyl (3-chloro-4-methoxyphenyl)carbamate (U.S. Patent 3860632) are known as synergistic agents for insecticidally active or acaricidally active substances. However, it has not been known or reported that they have fungicidal activity or are useful as fungicides.

The present invention also provides a combination composition comprising as active ingredients the N-phenylcarbamate (I) together with a benzimidazole, thiophanate and/or a cyclic imide fungicide, which is fungicidally effective against not only drug-sensitive fungi but also drug-resistant fungi, and hence particularly effective for the prevention of plant diseases. It also provides a method of controlling plant pathogenic fungi, including drug-resistant strains and drug-sensitive strains, by applying a fungicidally effective amount of the N-phenylcarbamate (I) to plant pathogenic fungi. It further provides novel members of the N-phenylcarbamates (I). It furthermore provides a process for producing the novel N-phenylcarbamates (I).

The N-phenylcarbamates (I) can be prepared by various procedures, among which typical examples are shown below:

### Procedures (a):-

The N-phenylcarbamate (I) is obtainable by reacting an aniline of the formula: 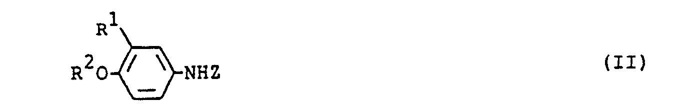 wherein R¹, R² and Z are each as defined above, with a chloroformate of the formula: 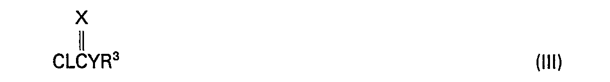 wherein R³, X and Y are each as defined above.

The reaction is usually carried out in the presence of an inert solvent (e.g. benzene, toluene, xylene, diethyl ether, tetrahydrofuran, dioxane, chloroform, carbon tetrachloride, ethyl acetate, pyridine, dimethylformamide). If desired, the reaction may be performed in the presence of a dehydrohalogenating agent (e.g. pyridine, triethylamine, diethylaniline, sodium hydroxide, potassium hydroxide, sodium hydride) to obtain the desired compound (I) in a high yield. The reaction may be carried out at a temperature in the range of from 0 to 150°C instantaneously or within 12 hours.

### Procedure (b):-

The N-phenylcarbamate (I) wherein Z is hydrogen may be obtained by reacting a phenyl isocyanate or isothiocyanate of the formula: 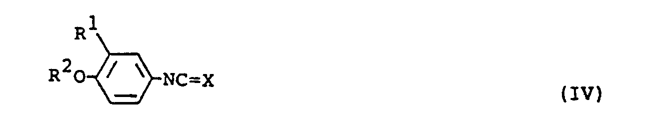 wherein R¹, R² and X are each as defined above, with an alcohol or thiol of the formula: 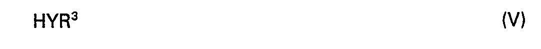 wherein R³ and Y are each as defined above.

The reaction is usually carried out in the absence or presence of an inert solvent (e.g. benzene, toluene, xylene, diethyl ether, tetrahydrofuran, dioxane, dimethylformamide, chloroform, carbon tetrachloride). If desired, a catalyst (e.g. triethylamine, diethylaniline, 1,4-diazabicyclo(2,2,2)octane) may be used. The reaction is normally carried out at a temperature in the range of from 0 to 50°C instantaneously or within 12 hours.

### Procedure (c):-

The N-phenylcarbamate (I) wherein Z is not hydrogen may be obtained by reacting an N-phenylcarbamate of the formula: 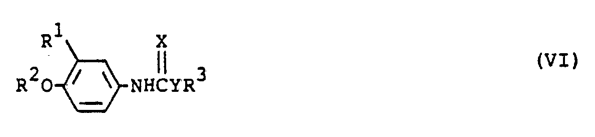 wherein R¹, R2, R³, X and Y are each as defined above, with a halide of the formula: 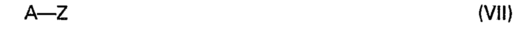 wherein Z is as defined above except it is other than hydrogen and A is a halogen atom (e.g. chlorine, bromine).

The reaction is usually carried out in an inert sovlent (e.g. benzene, toluene, xylene, diethyl ether, tetrahydrofuran, dioxane, chloroform, carbon tetrachloride, ethyl acetate, pyridine, dimethylformamide). If desired, the reaction may be performed in the presence of a dehydrohalogenating agent (e.g. pyridine, triethylamine, diethylaniline, sodium hydroxide, potassium hydroxide, sodium hydride) and a catalyst (e.g. tetrabutylammonium bromide) to obtain the desired compound (I) in a high yield. The reaction may be carried out at a temperature in the range of from 0 to 150°C instantaneously or within 12 hours.

The N-phenylcarbamates (I) arefungicidally effective against a wide scope of plant pathogenic fungi, of which examples are as follows: Podosphaera leucotricha, Venturia inaequalis, Mycosphaerella pomi, Marssonina mali and Sclerotinia mali of apple, Phyllactinia kakicola and Gloeosporium kaki of persimmon, Cladosporium carpophilum and Phomopsis sp. of peach, Cercospora viticola, Uncinula necator, Elsinoe ampelina and Glomerella cingulata of grape, Cercospora beticola of sugarbeet, Cercospora arachidicola and Cercospora personata of peanut, Erysiphe graminis f. sp. hordei, Cercosporella herpotrichoides and Fusarium nivale of barley, Erysiphe graminis f. sp. tritici of wheat, Sphaerotheca fuliginea and Cladosporium cucumerinum of cucumber, Cladosporium fulvum of tomato, Corynespora melongenae of eggplant, Sphaerotheca humuli, Fusarium oxysporum f. sp. fragariae of strawberry, Botrytis allii of onion, Cercospora apii of celery, Phaeoisariopsis griseola of kidney bean, Erysiphe cichoracearum of tobacco, Diplocarpon rosae of rose, Elsinoe fawcetti, Penicillium italicum, Penicillium digitatum of orange, Botrytis cinerea of cucumber, eggplant, tomato, strawberry, pimiento, onion, lettuce, grape, orange, cyclamen, rose or hop, Sclerotinia sclerotiorum of cucumber, eggplant, pimiento, lettuce, celery, kidney bean, soybean, azuki bean, potato or sunflower, Sclerotinia cinerea of peach or cherry, Mycosphaerella melonis of cucumber or melon, etc. The N-phenylcarbamates of formula (I) are highly effective in controlling the drug-resistant strains of these fungi.

The N-phenylcarbamates (I) are also fungicidally effective against fungi sensitive to the known fungicides as well as fungi against which the known fungicides are ineffective. Examples of such fungi are pyricularia oryzae, Pseudoperonospora cubensis, Plasmopara viticola, Phytophthora infestans, etc.

Advantageously, the N-phenylcarbamates (I) are low toxicity and have little detrimental effect on mammals and fish. They may be applied to agricultural fields without causing any material toxicity to important crop plants.

In view of their excellent fungicidal properties, preferred are the compounds of the formula (I) wherein R¹ is a C₁-C₄ alkyl group, a C₃-C₄ alkenyl group, an ethynyl group, a cyano group, a cyanomethyl group, a chloromethyl group, a 2-chloropropyl group, a methoxymethyl group, an ethoxymethyl group, a methoxycarbonyl group, an ethoxycarbonyl group, a dimethoxymethyl group, a methylenedioxymethyl group, an acetyl group, a propionyl group, a methoxyiminomethyl group or an N-ethylcarbamoyl group; R² is a C₁-C₃ alkyl group, an allyl group, a propargyl group, a difluoromethyl group, a 2-chloroethyl group, a 2-fluoroethyl group, a 2,2,2-trifluoroethyl group, a 2-methoxyethyl group or a 2-cyclopropylethyl group; R³ is a C₁-C₈ alkyl group, a C₃-C₈ alkenyl group, a C₃-C₈ alkynyl group a cyclobutyl group, a 4-chloro-2-butenyl group, a 4-chloro-2-butynyl group, a 1-phenylethyl group, a 2-fluoroethyl group, a 1-(chloromethyl)ethyl group, a 2-cyanoethyl group, a 1-(methoxymethyl)ethyl group, a 2-chloro-1-(methoxymethyl)ethyl group, a 1-(allyloxymethyl)ethyl group, a 1-(2-chloroethoxymethyl)ethyl group, a 1-(phenoxymethyl)ethyl group, a 1-(benzyloxymethyl)ethyl group or a 1-(cyclopropyl)ethyl group; X and Y are, each independently an oxygen atom or a sulfur atom; and Z is a hydrogen atom, a methyl group, an allyl group, a propargyl group, a cyanomethyl group, an ethoxycarbonylmethyl group, an acetyl group, a 1-methyl- butanoyl group a cyclopropanecarbonyl group, a chloroacetyl group, a benzoyl group, a 2-chlorobenzoyl group or a methylsulfonyl group. Particularly preferred are the compounds of the formula (I) wherein R¹ is an ethyl group, a propyl group, an allyl group, a methoxymethyl group, an ethoxymethyl group or an ethoxycarbonyl group; R² is an ethyl group, a propyl group, an allyl group, a propargyl group or a 2-chloroethyl group; R' is a methyl group, an ethyl group, an isopropyl group, a sec-butyl group, a 1-methyl-2-propenyl group, a 1-methyl-2-propynyl group, a 4-chloro-2-butynyl group, a 1-phenylethyl group, a 2-fluoroethyl group, a 1-(chloromethyl)ethyl group, a 2-cyanoethyl group or a 1-(methoxymethyl)ethyl group; X is an oxygen atom; Y is an oxygen atom or a sulfur atom; and Z is a hydrogen atom, an acetyl group or a benzoyl group.

Most preferred are the following: isopropyl N-(3-ethyl-4-ethoxyphenyl)carbamate, isopropyl N-(3-propyl-4-ethoxyphenyl)carbamate, 1-methyl-2-propynyl N-(3-propyl-4-ethoxyphenyllcarbamate, isopropyl N-(3-methoxymethyl-4-ethoxyphenyllcarbamate, sec-butyl N-(3-methoxymethyl-4-ethoxyphenyl)-carbamate, 1-(methoxymethyl)ethyl N-(3-methoxymethyl-4-ethoxyphenyl)carbamate, isopropyl N-(3- ethoxymethyl-4-ethoxyphenyl)carbamate, etc.

Some typical examples of the procedures for preparation of the N-phenylcarbamates (1) are illustratively shown in the following examples.

### Example 1

### Preparation of isopropyl N-(3-ethyl-4-ethoxyphenyl)carbamate according to Procedure (a):-

3-Ethyl-4-ethoxyaniline (1.7 g) and diethylaniline (1.5 g) were dissolved in toluene (20 ml). To the resultant solution was dropwise added isopropyl chloroformate (1.2 g) in 5 minutes under ice-cooling. After being allowed to stand at room temperature for 12 hours, the reaction mixture was poured into ice water and extracted with toluene. The extract was washed with water, dried over magnesium sulfate and concentrated under reduced pressure. The residue was purified by silica gel chromatography using toluene as the eluent to give isopropyl N-(3-ethyl-4-ethoxyphenyl)carbamate (Compound No. 50) (2.2 g) in a yield of 88%. M.P., 81-82°C.

Elementary analysis: Calcd. for C₁₄H₂₁NO₃: C, 66.90%; H, 8.42%; N, 5.57%. Found: C, 66.97%; H, 8.33%; 5.62%.

### Example 2

### Preparation of isopropyl N-(3-methoxymethyl-4-ethoxyphenyl)carbamate according to Procedure (b):-

A mixture of 3₋methoxymethyl-4-ethoxyaniline (1.8 g) in toluene (20 ml) was dropwise added to a toluene solution containing 10 g of phosgene at 10 to 20°C. The resulting mixture was gradually heated and, after being refluxed for 30 minutes, cooled to room temperature. The solvent was removed by distillation under reduced pressure to give 3-methoxymethyl-4-ethoxyphenyl isocyanate. The obtained crude substance was added to an isopropanol solution (20 ml) containing triethylamine (1 g). The resultant mixture was allowed to stand at room temperature for 12 hours, poured into ice-water and extracted with toluene. The extract was washed with water, dried over magnesium sulfate and concentrated under reduced pressure. The residue was purified by silica gel chromatography using toluene as the eluent to give isopropyl N-(3-methoxymethyl-4-ethoxyphenyl)carbamate (Compound No. 77) (2.4 g) in a yield of 90% (calculated from the staring 3-methoxymethyl-4-ethoxyaniline). M.P., 41-42.5°C.

Elementary analysis: Calcd. for C₁₄H₂₁NO₄: C, 62.90%; H, 7.92%; N, 5.27%. Found: C, 62.66%; H, 7.96%; N, 5.41%.

### Example 3

### Preparation of isopropyl N-benzoyl-N-(3-methoxymethyl-4-ethoxyphenyl)carbamate according to Procedure (c):-

Isopropyl N-(3-methoxymethyl-4-ethoxyphenyl)carbamate (2.7 g) was dissolved in dimethylformamide (50 ml), and sodium hydride dispersion (50%, 0.5 g) was added thereto. The mixture was heated at 60°C for 15 minutes, treated with benzoyl chloride (1.4 g) and heated for 30 minutes. The reaction mixture was poured into ice-water and extracted with ether. The extract was washed with sodium bicarbonate solution and saturated brine, dried over anhydrous magnesium sulfate and concentrated under reduced pressure. The residue was purified by silica gel chromatography using hexane-acetone as the eluent to give isopropyl N-benzoyl-N-(3-methoxymethyl-4-ethoxyphenyl)carbamate (Compound No. 134) (2.6 g) in a yield of 68%. M.P., 87-89°C.

Elementary analysis: Calcd. for C₂₁H₂₅NO₅: C, 67.90%; H, 6.78%; N, 3.77%. Found: C, 67.70%; H, 6.81 %; N, 3.80%.

According to either one of the above Procedures (a), (b) or (c), the N-phenylcarbamates of the formula (I) as shown in Table 1 can be prepared:

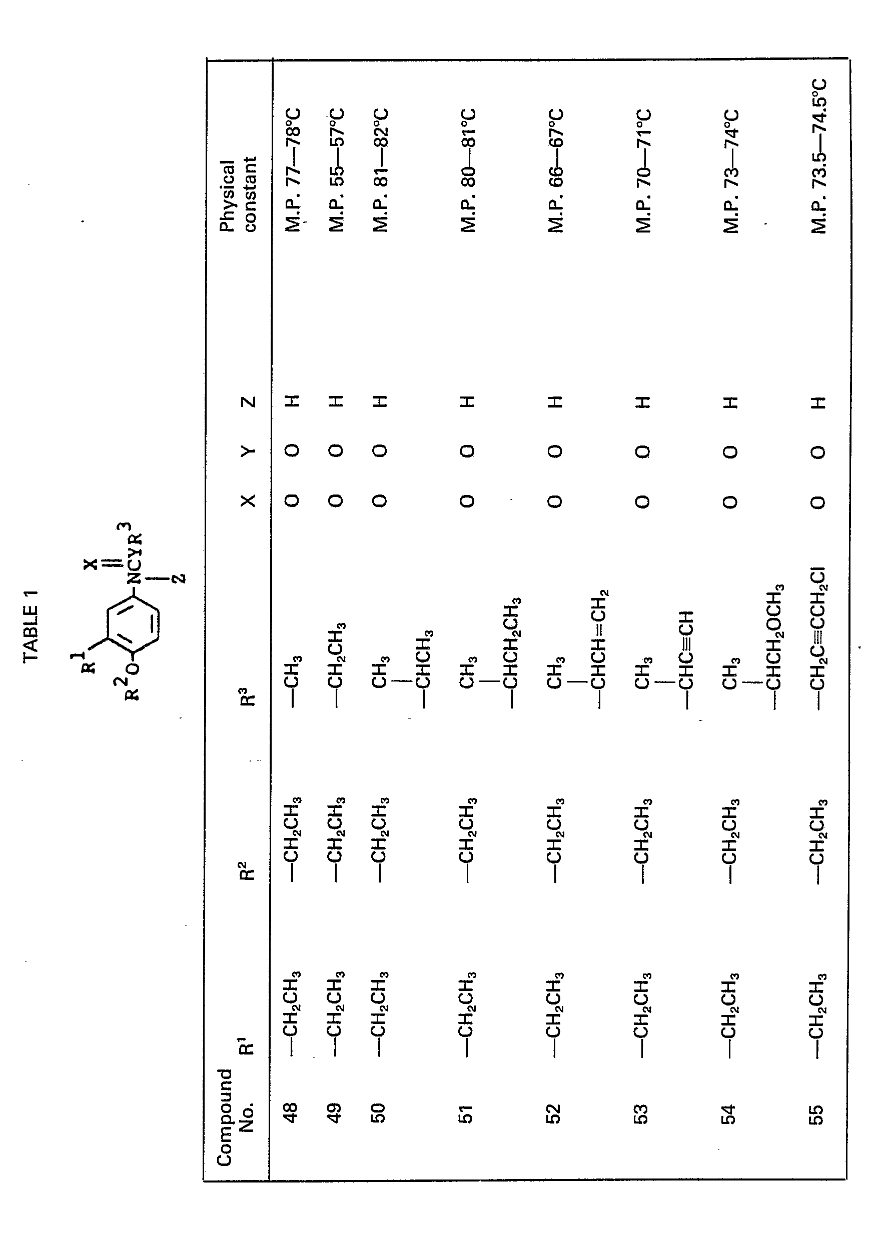

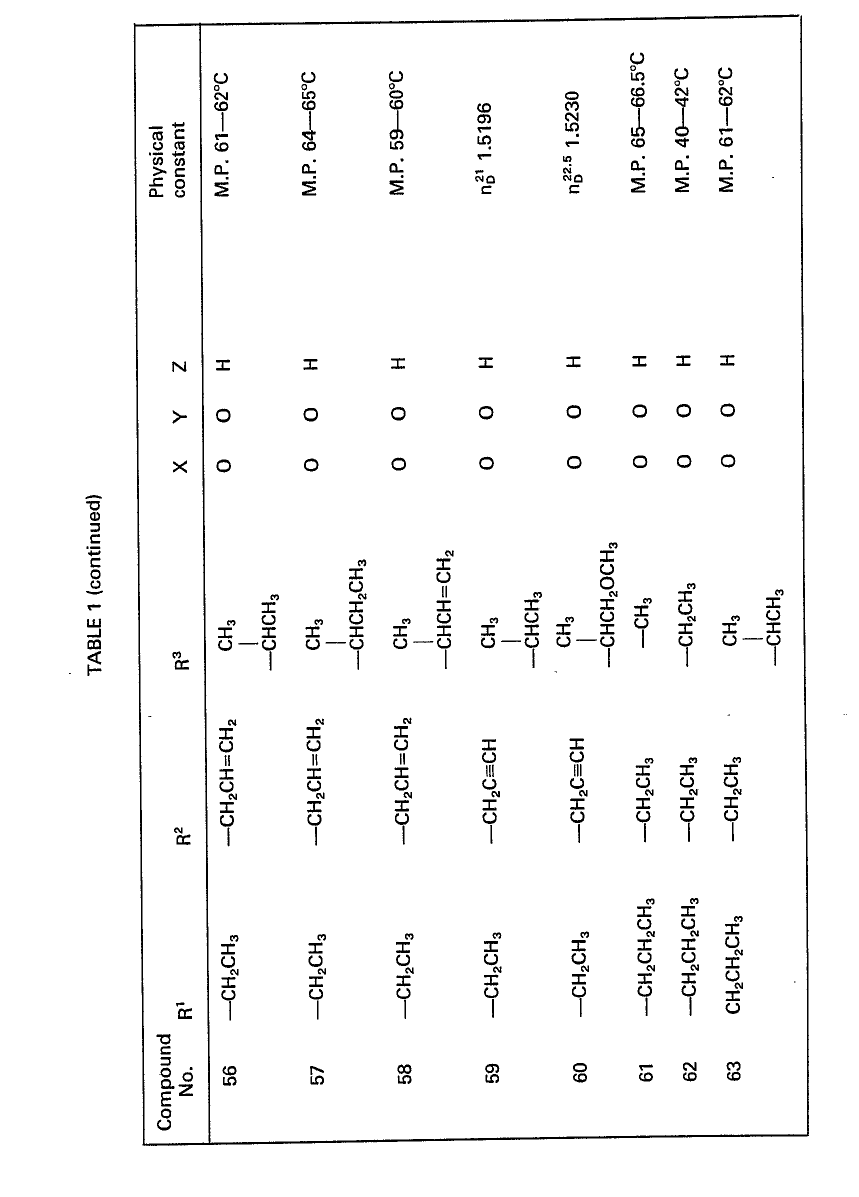

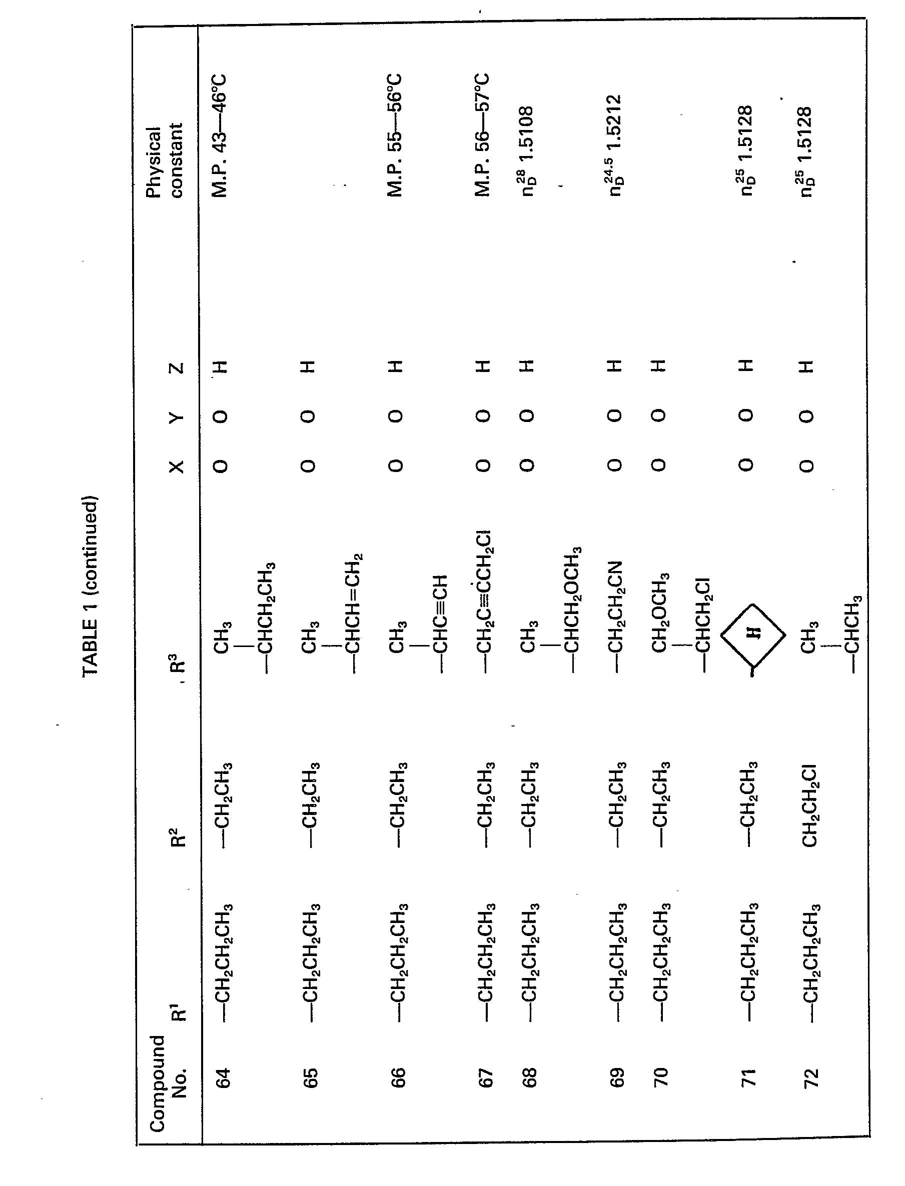

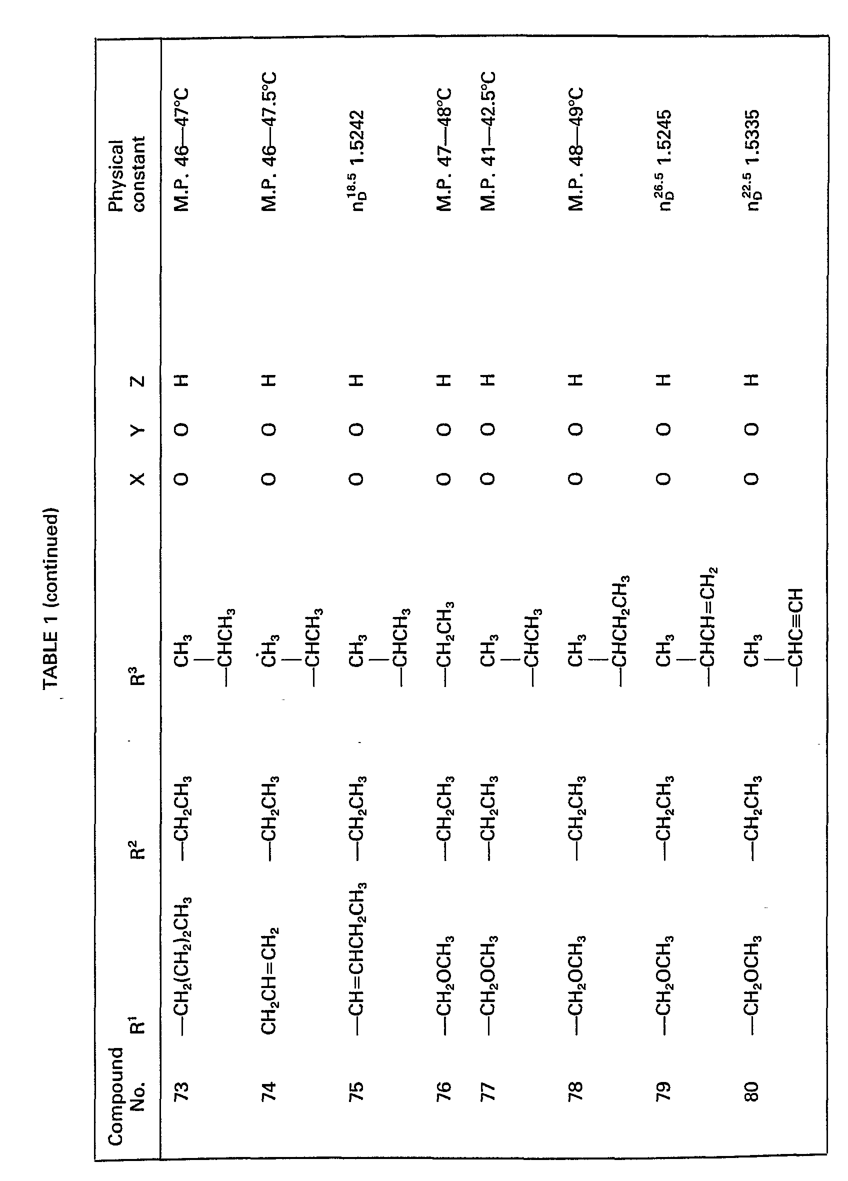

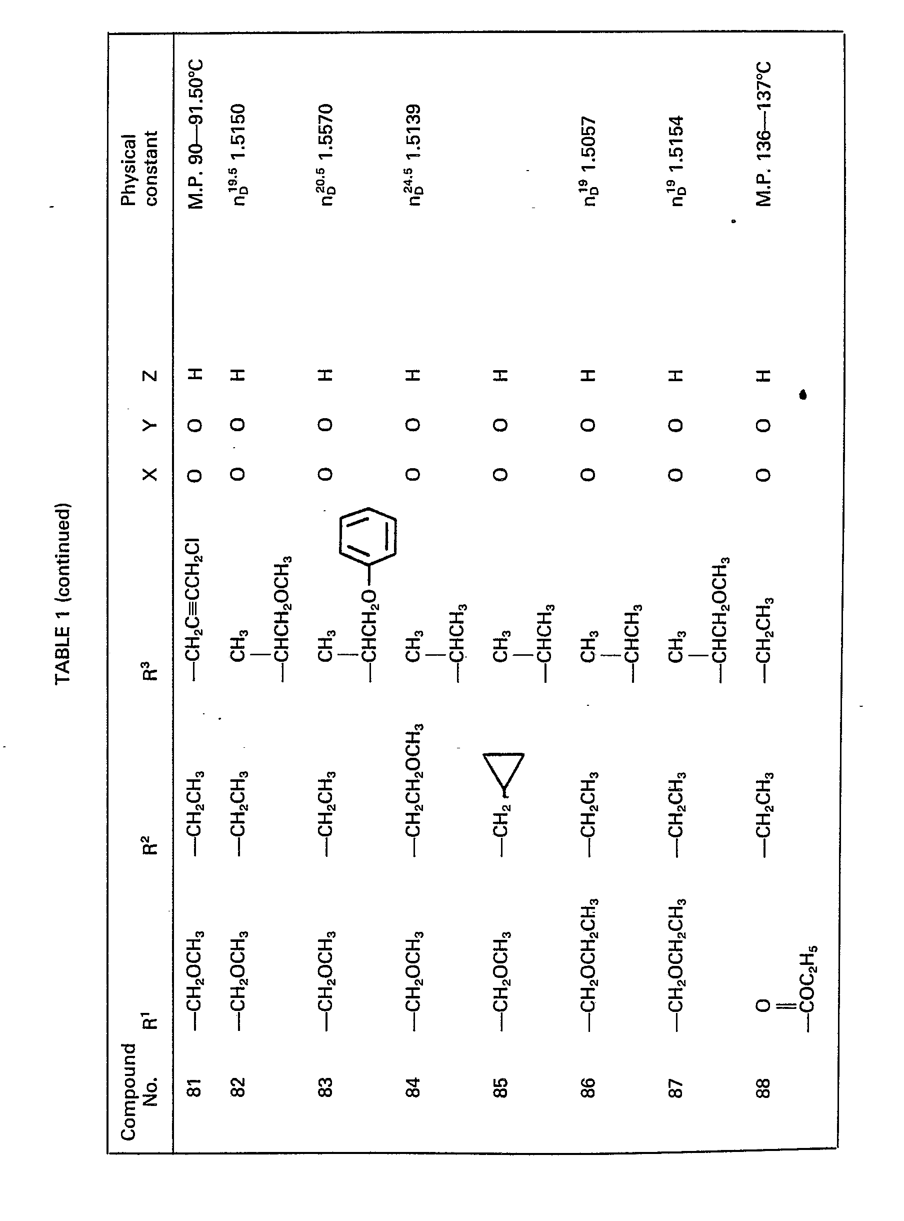

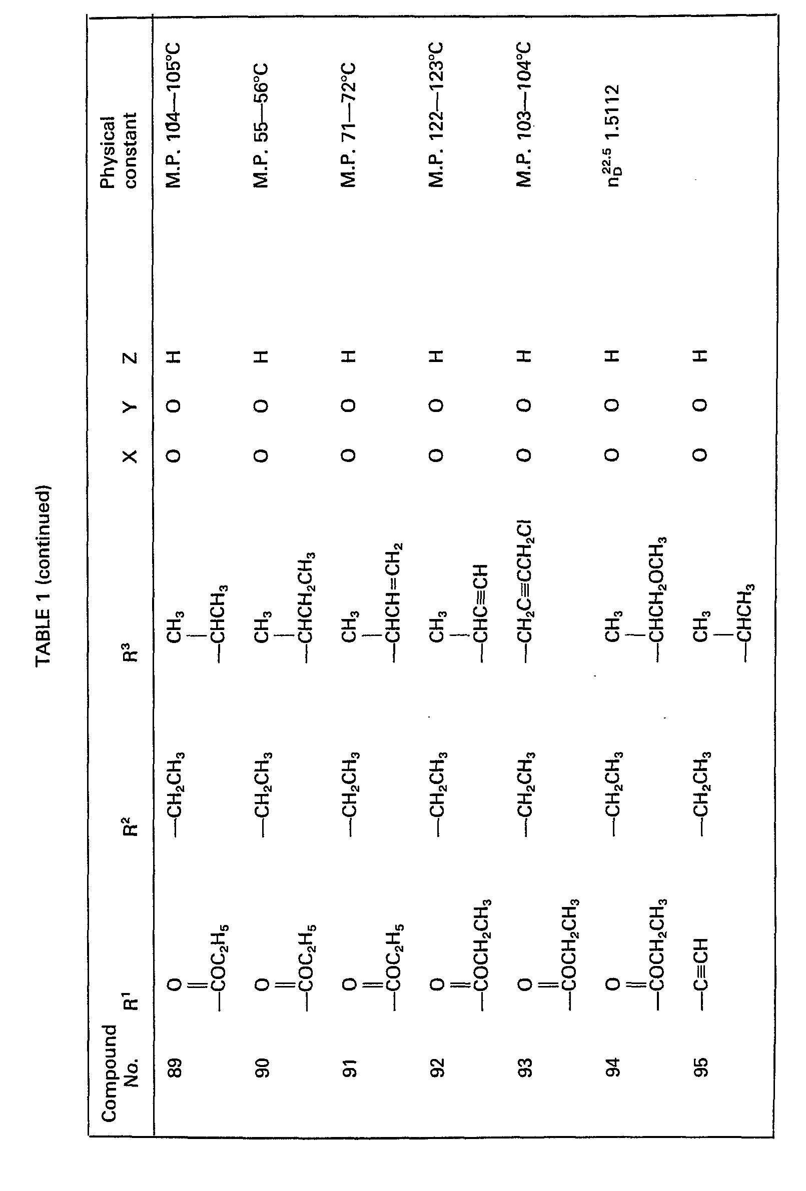

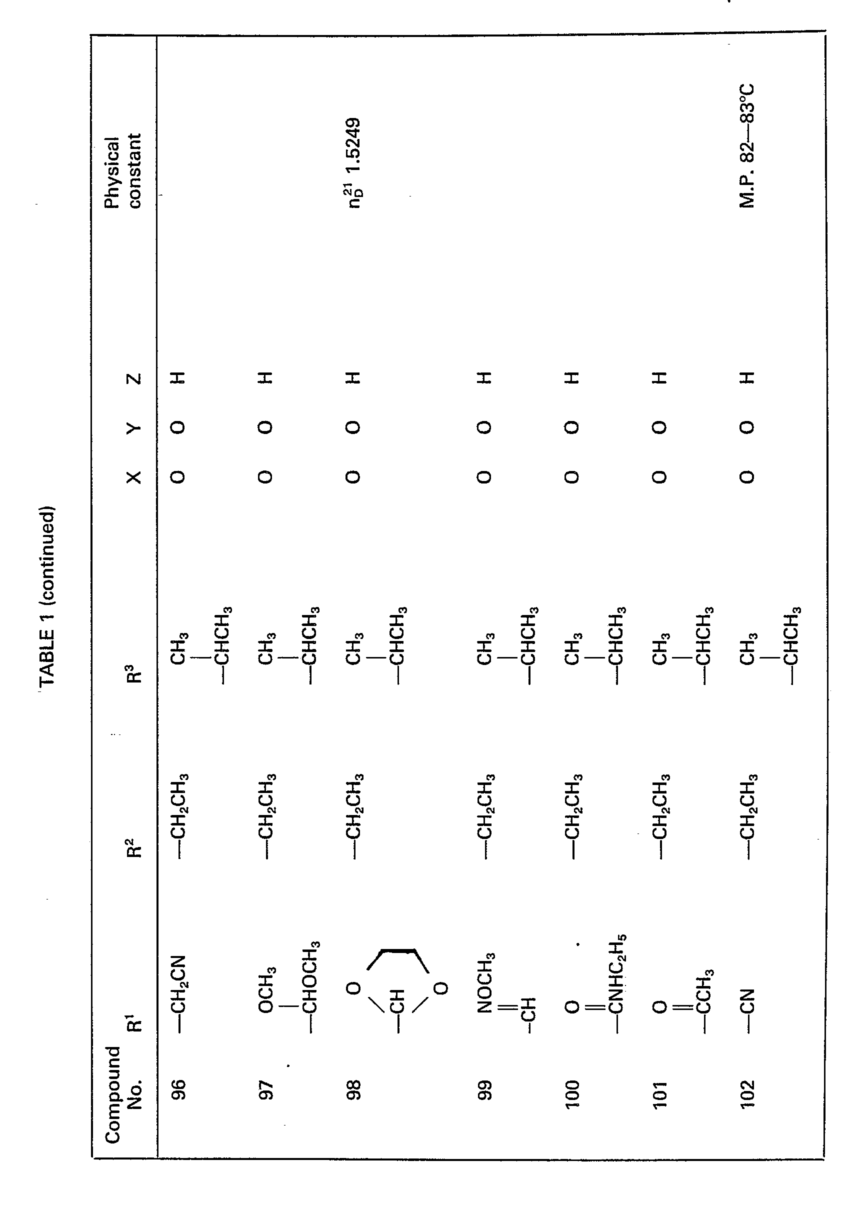

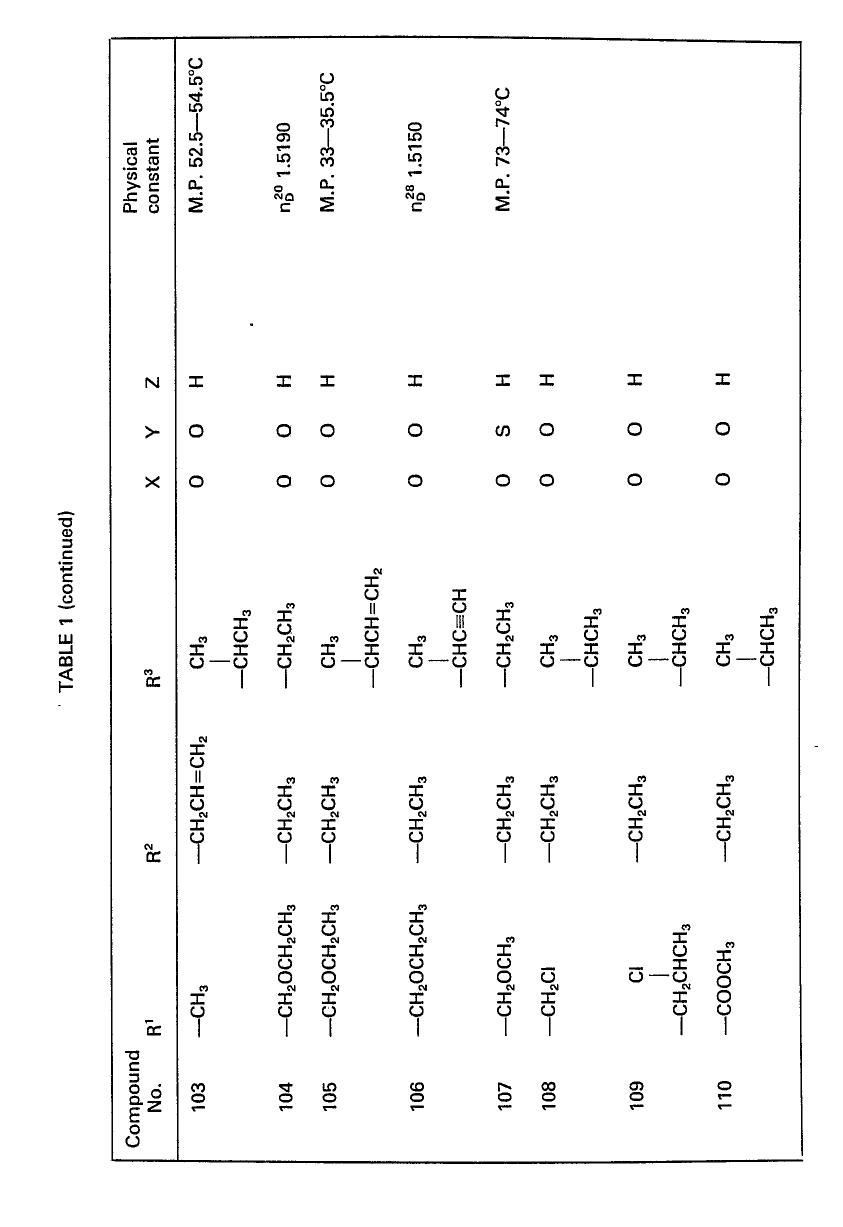

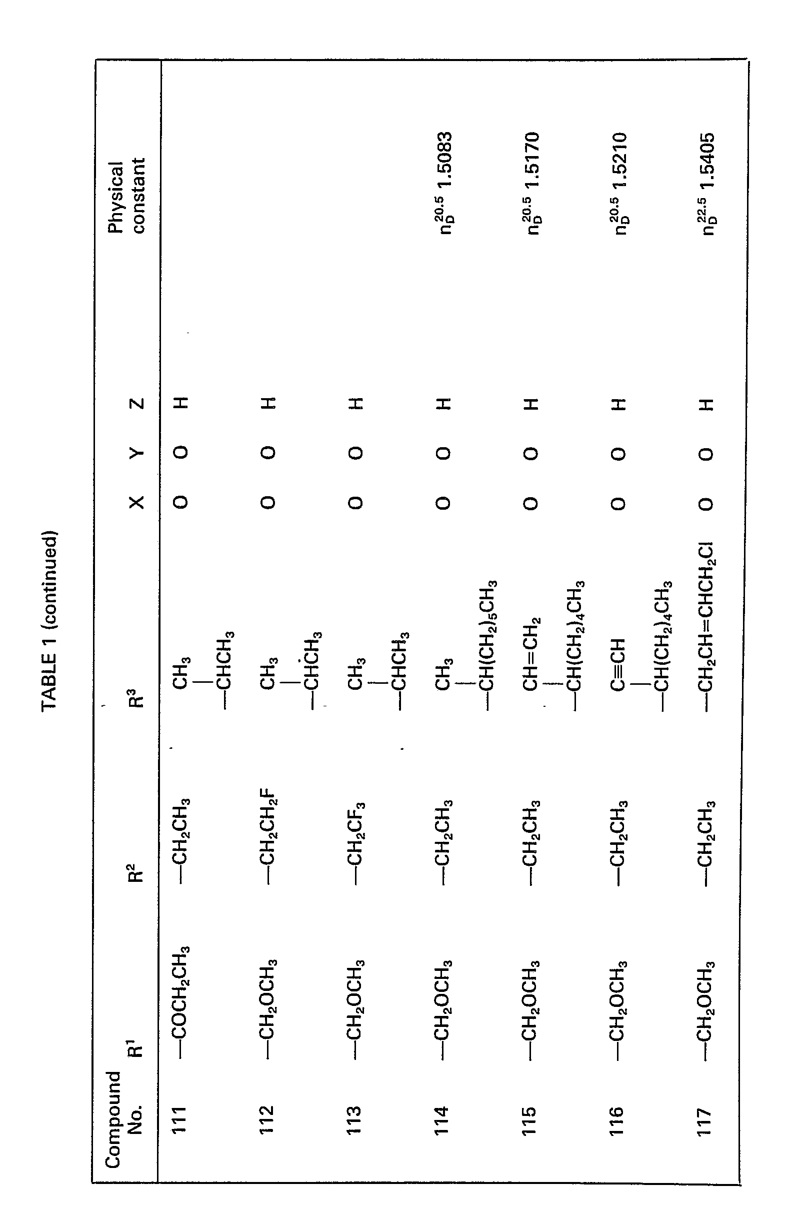

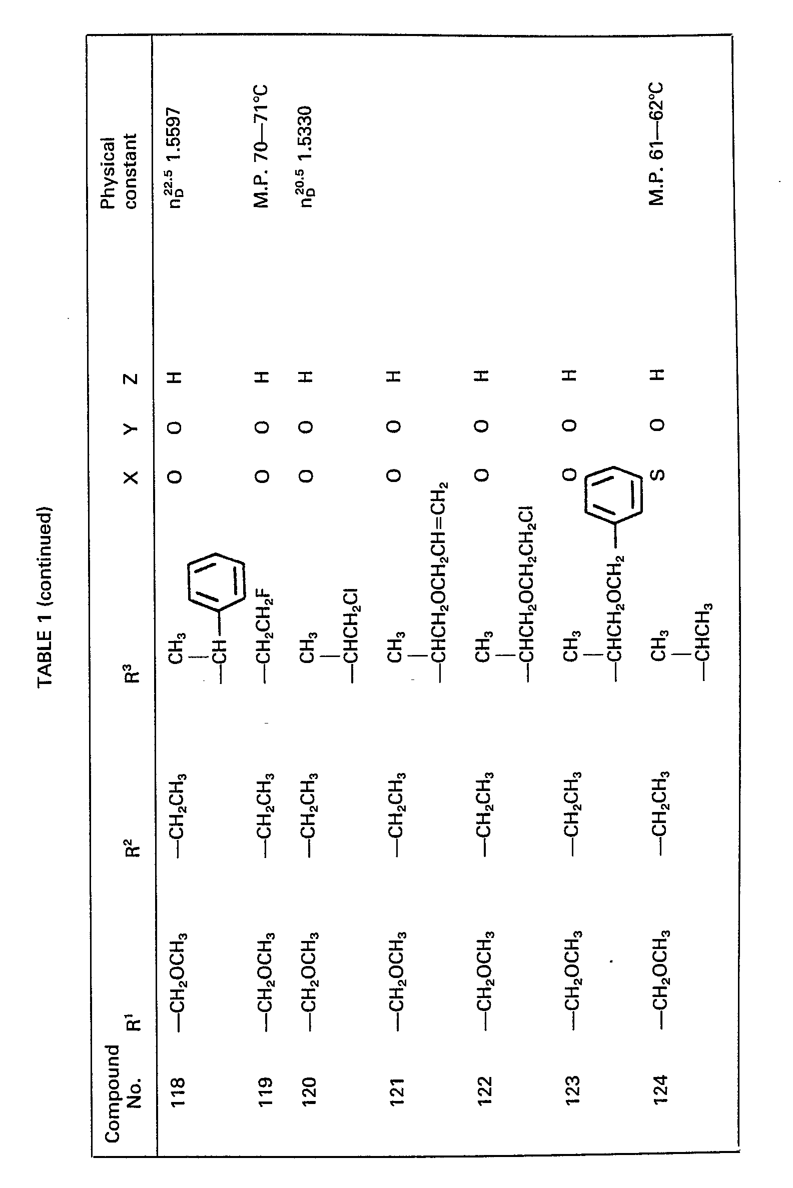

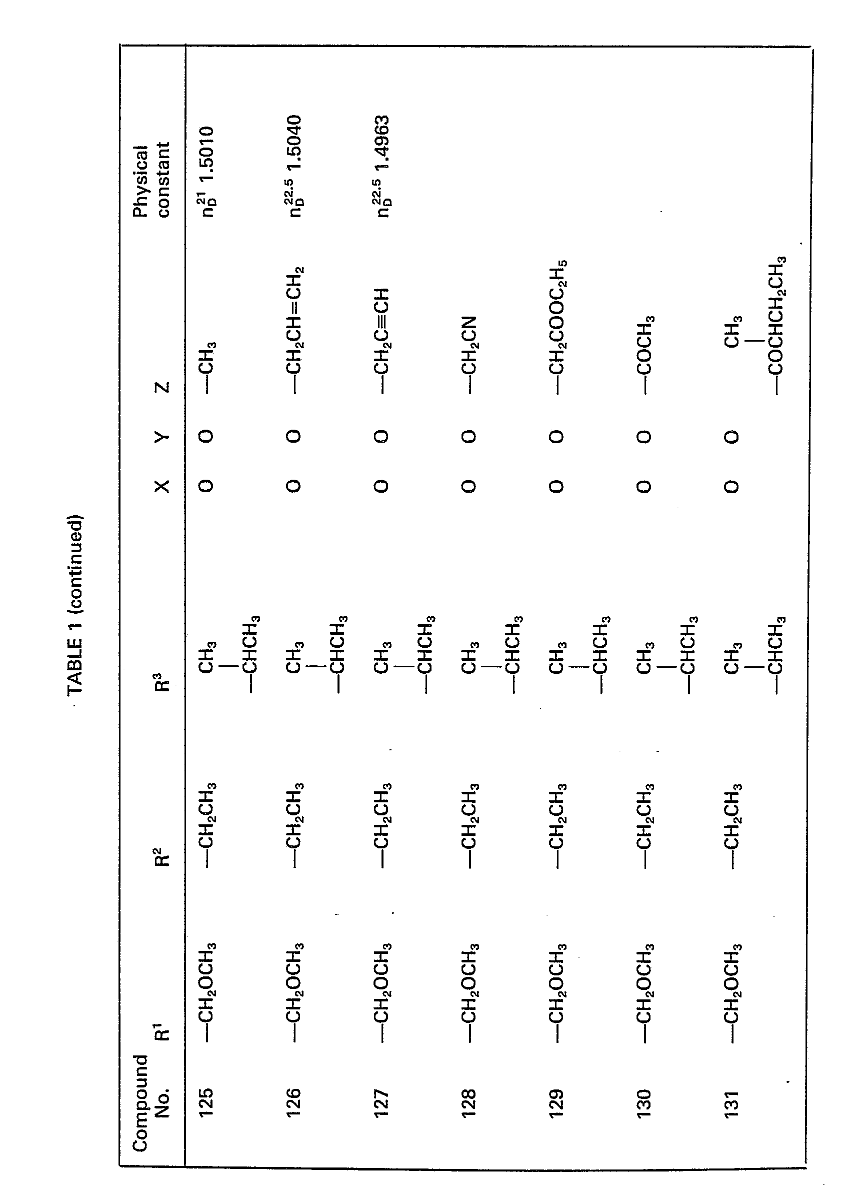

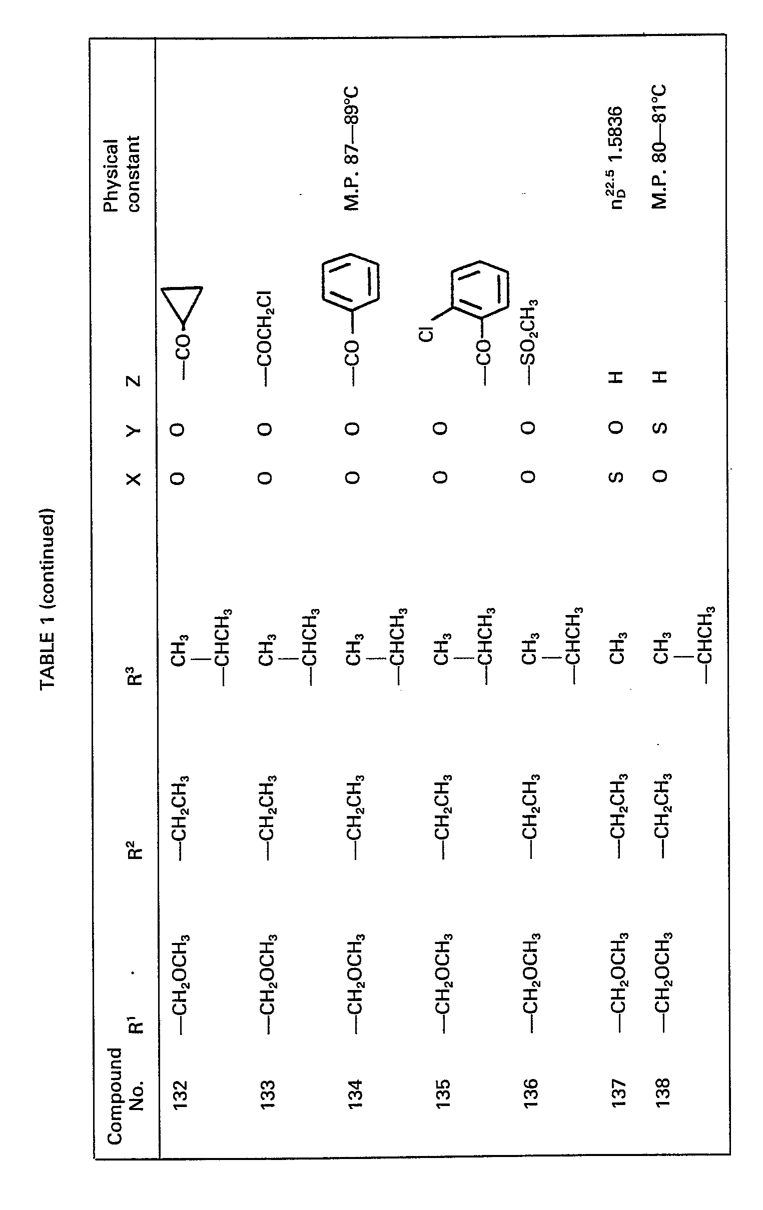

In the practical usage of the N-phenylcarbamates (1) as a fungicide, they may be applied as such or in a preparation form such as dusts, wettable powders, oil sprays, emulsifiable concentrates, tablets, granules, fine granules, aerosols or flowables. Such preparation form can be prepared in a conventional manner by mixing at least one of the N-phenylcarbamates (I) with an appropriate solid or liquid carrier(s) or diluent(s) and, if necessary, an appropriate adjuvant(s) (e.g. surfactants, adherents, dispersants, stabilizers) for improving the dispersibility and other properties of the active ingredient.

Examples of the solid carriers or diluents are botanical materials (e.g. flour, tobacco stalk powder, soybean powder, walnut-shell powder, vegetable powder, saw dust, bran, bark powder, cellulose powder, vegetable extract residue), fibrous materials (e.g. paper, corrugated cardboard, old rags), synthetic plastic powders, clays (e.g. kaolin, bentonite, fuller's earth), talcs, other inorganic materials (e.g. pyrophyllite, sericite, pumice, sulfur powder, active carbon) and chemical fertilizers (e.g. ammonium sulfate, ammonium phosphate, ammonium nitrate, urea, ammonium chloride).

Examples of the liquid carriers or diluents are water, alcohols (e.g. methanol, ethanol), ketones (e.g. acetone, methylethylketone), ethers (e.g. diethyl ether, dioxane, cellosolve, tetrahydrofuran), aromatic hydrocarbons (e.g. benzene, toluene, xylene, methyl naphthalene), aliphatic hydrocarbons (e.g. gasoline, kerosene, lamp oil), esters, nitriles, acid amides (e.g. dimethylformamide, dimethylacetamide), halogenated hydrocarbons (e.g. dichloroethane, carbon tetrachloride), etc.

Examples of the surfactants are alkyl sulfuric esters, alkyl sulfonates, alkylaryl sulfonates, polyethylene glycol ethers, polyhydric alcohol esters, etc. Examples of the adherents and dispersants may include casein, gelatin, starch powder, carboxymethyl cellulose, gum arabic, alginic acid, lignin, bentonite, molasses, polyvinyl alcohol, pine oil and agar. As the stabilizers, there may be used PAP (isopropyl acid phosphate mixture), tricresyl phosphate (TCP), tolu oil, epoxydised oil, various surfactants, various fatty acids and their esters, etc.

The foregoing preparations generally contain at least one of the N-phenylcarbamates (I) in a concentration of about 1 to 95% by weight, preferably of 2.0 to 80% by weight. By using the preparations, the N-phenylcarbamates (I) are generally applied in such amounts as 2 to 100 g per 10 are.

When only the drug-resistant strains of phytopathogenic fungi are present, the N-phenylcarbamates (I) may be used alone. However, when the drug-sensitive strains are present together with the drug-resistant strains, their alternate use with benzimidazole, thiophanate and/or dicarboxamide fungicides or their combined use with benzimidazole, thiophanate and/or cyclic imide fungicide is favorable. In such alternate or combined use, each active ingredient may be employed as such or in conventional agricultural preparation forms. In case of the combined use, the weight proportion of the N-phenylcarbamates (I) and the benzimidazole, thiophanate and/or cyclic imide fungicide may be from about 1:0.1 to 1:10.0.

Typical examples of the benzimidazole, thiophanate and dicarboximide fungicides are shown in Table 2.

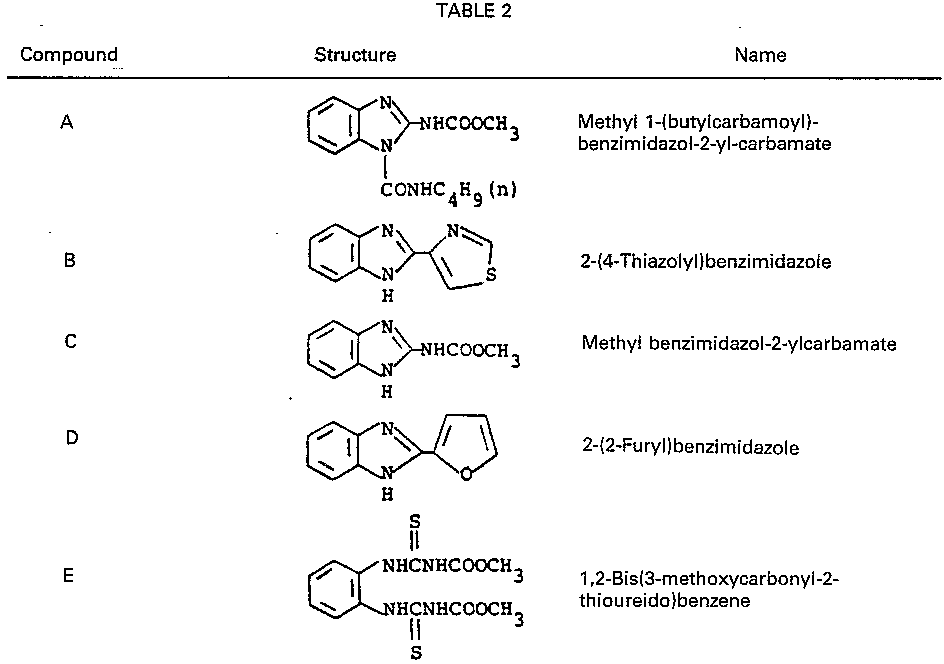

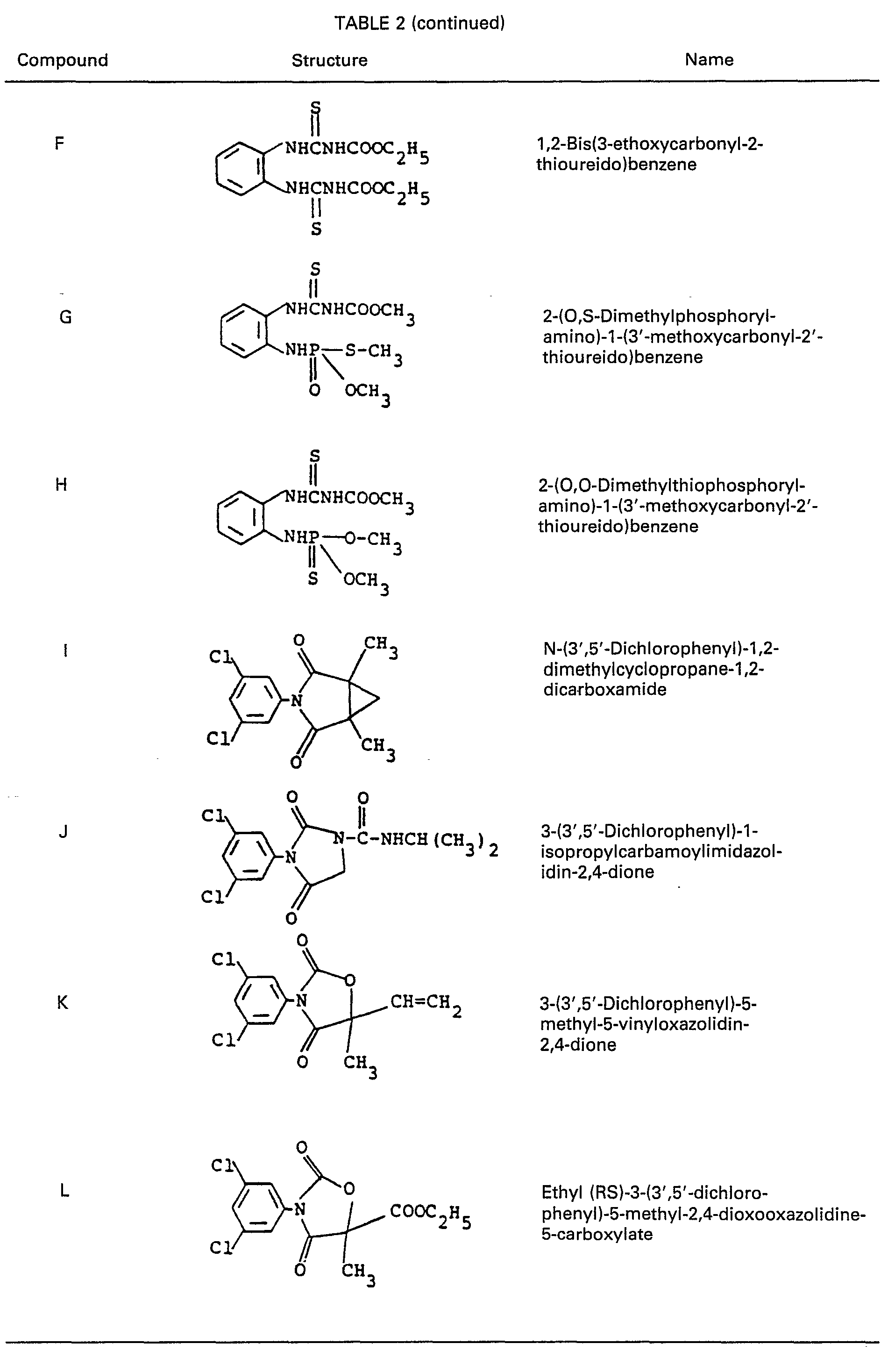

Besides, the N-phenylcarbamates (I) may be also used in admixture with other fungicides, herbicides, insecticides, miticides, fertilizers, etc.

When the N-phenylcarbamates (I) are used as fungicides, they may be applied in such amounts as 2 to 100 grams per 10 are. However, this amount may vary depending upon formulation forms, application times, application methods, application sites, diseases, crops and so on, and therefore, they are not limited to said particular amounts.

Some practical embodiments of the fungicidal composition according to the invention are illustratively shown in the following Examples wherein % and part(s) are by weight.

### Formulation Example 1

Fifty parts of Compound No. 89, 45 parts of diatomaceous earth, 2.5 parts of calcium alkylbenzenesulfonate as a wetting agent and 2.5 parts of calcium ligninsulfonate as a dispersing agent were mixed while being powdered to obtain a wettable powder formulation containing 50% of the active ingredient.

### Formulation Example 2

One part of Compound No. 77, 1 part of Compound No. 1, 88 parts of clay and 10 parts of talc were thoroughly pulverized and mixed together to obtain a dust formulation containing 2% of the active ingredient.

### Formulation Example 3

Twenty parts of Compound No. 63,10 parts of Compound J, 45 parts of diatomaceous earth, 20 parts of white carbon, 3 parts of sodium laurylsulfate as a wetting agent and 2 parts of calcium ligninsulfonate as a dispersing agent are mixed while being powdered to obtain a wettable powder formulation containing 30% of the active ingredient.

### Formulation Example 4

Ten parts of Compound No. 76, 40 parts of Compound No. B, 45 parts of diatomaceous earth, 2.5 parts of calcium alkylbenzenesulfonate as a wetting agent and 2.5 parts of calcium ligninsulfonate as a dispersing agent were mixed while being powdered to obtain a wettable powder formulation containing 50% of the active ingredient.

### Formulation Example 5

Twenty-five parts of Compound No. 54, 50 parts of Compound No. I, 18 parts of diatomaceous earth, 3.5 parts of calcium alkylbenzenesulfonate as a wetting agent and 3.5 parts of calcium ligninsulfonate as a dispersing agent were mixed while being powdered to obtain a wettable powder formulation containing 75% of the active ingredient.

### Formulation Example 6

Twenty parts of Compound No. 50,30 parts of Compound No. A, 40 parts of powdery sucrose, 5 parts of white carbon, 3 parts of sodium laurylsulfate as a wetting agent were mixed while being powdered to obtain a wettable powder formulation containing 50% of the active ingredient.

Typical test data indicating the excellent fungicidal activity of the N-phenylcarbamates (I) are shown below. The compounds used for comparison are as follows:

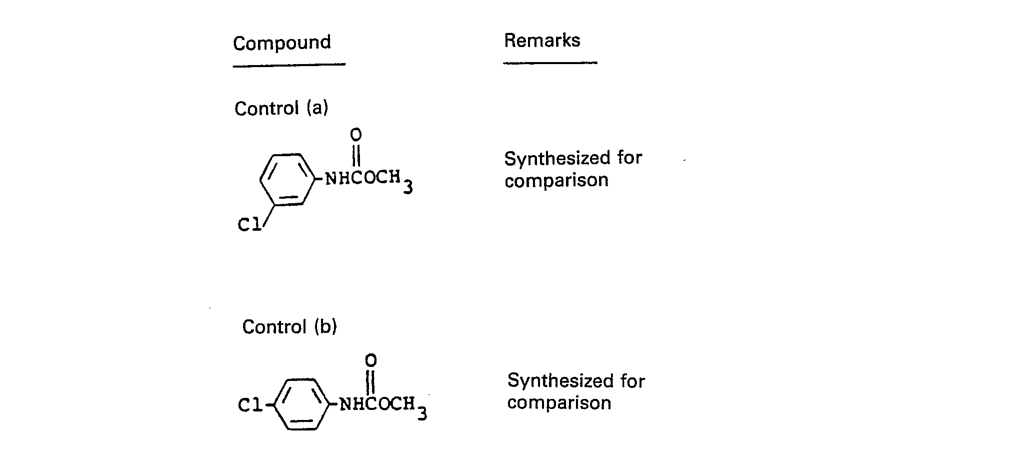

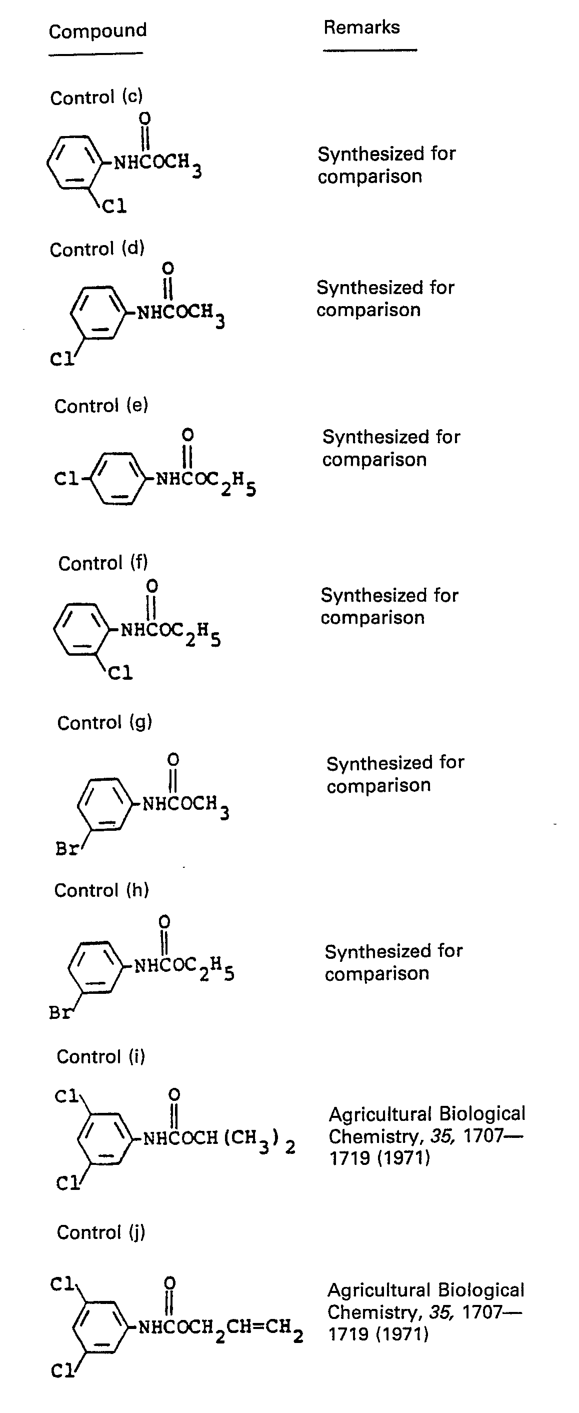

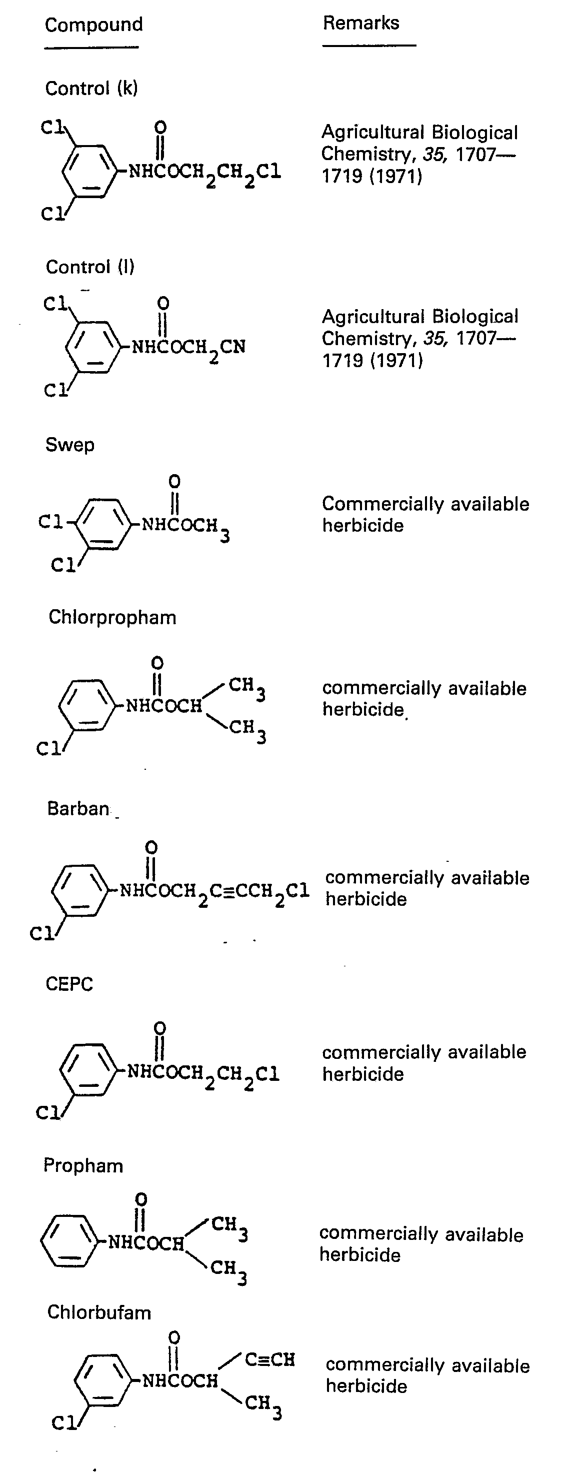

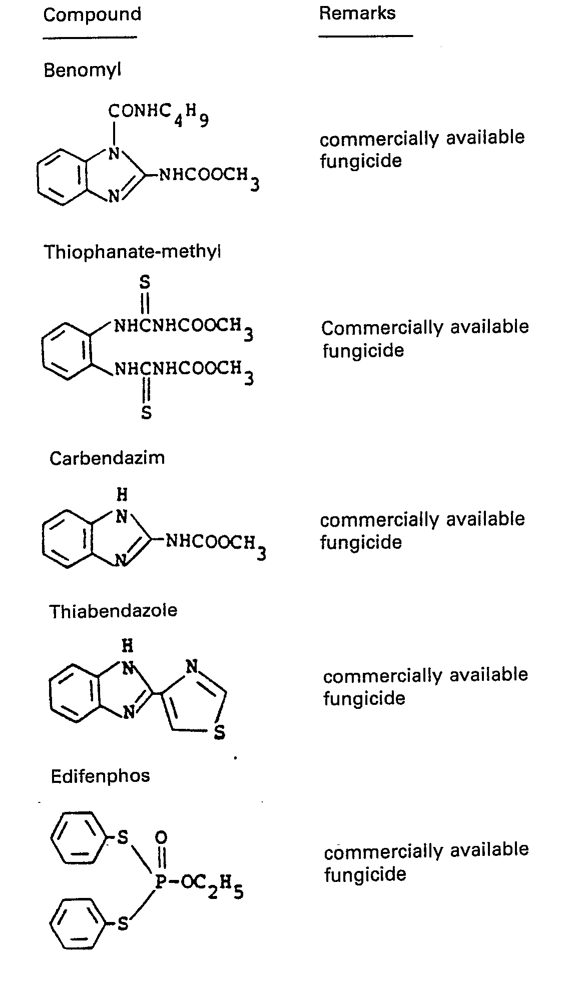

### Experiment 1

### Protective activity test on powdery mildew of cucumber (Sphaerotheca fuliginea):-

A flower pot of 90 ml volume was filed with sandy soil, and seeds of cucumber (var: Sagami-hanjiro) were sowed therein. Cultivation was carried out in a greenhouse for 8 days. Onto the resulting seedlings having cotyledons, the test compound formulated in emulsifiable concentrate or wettable powder and diluted with water was sprayed at a rate of 10 ml per pot. Then, the seedlings were inoculated with a spore suspension of the drug-resistant or drug-sensitive strain of Sphaerotheca fuliginea by spraying and further cultivated in the greenhouse. Ten days thereafter, the infectious state of the plants was observed. The degree of damage was determined in the following manner, and the results are shown in Table 3.

The leaves examined were measured for a percentage of infected area and classified into the corresponding disease indices, 0, 0.5, 1, 2, 4:

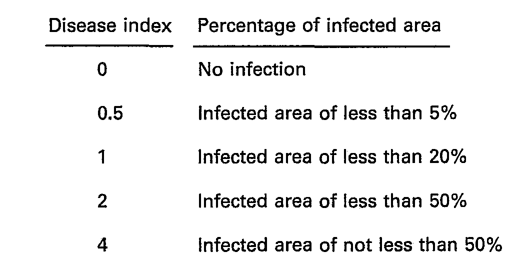

The disease severity was calculated according to the following equation:

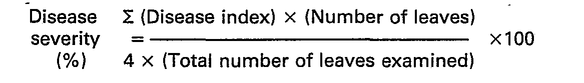

The prevention value was calculated according to the following equation:

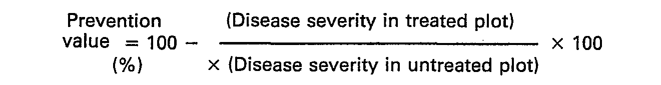

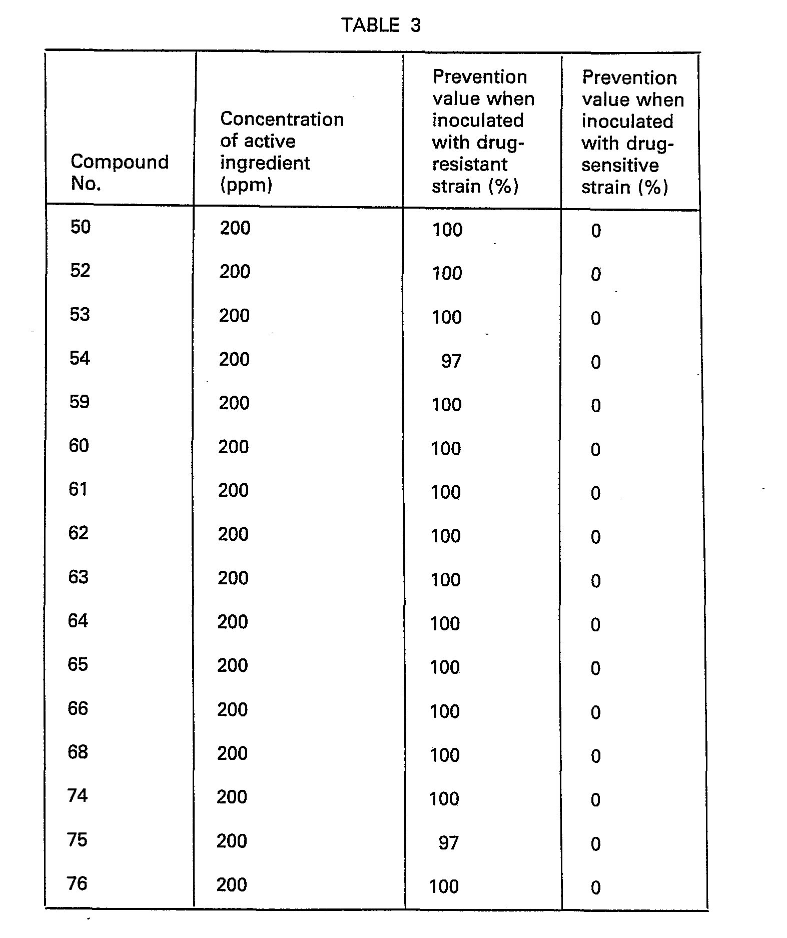

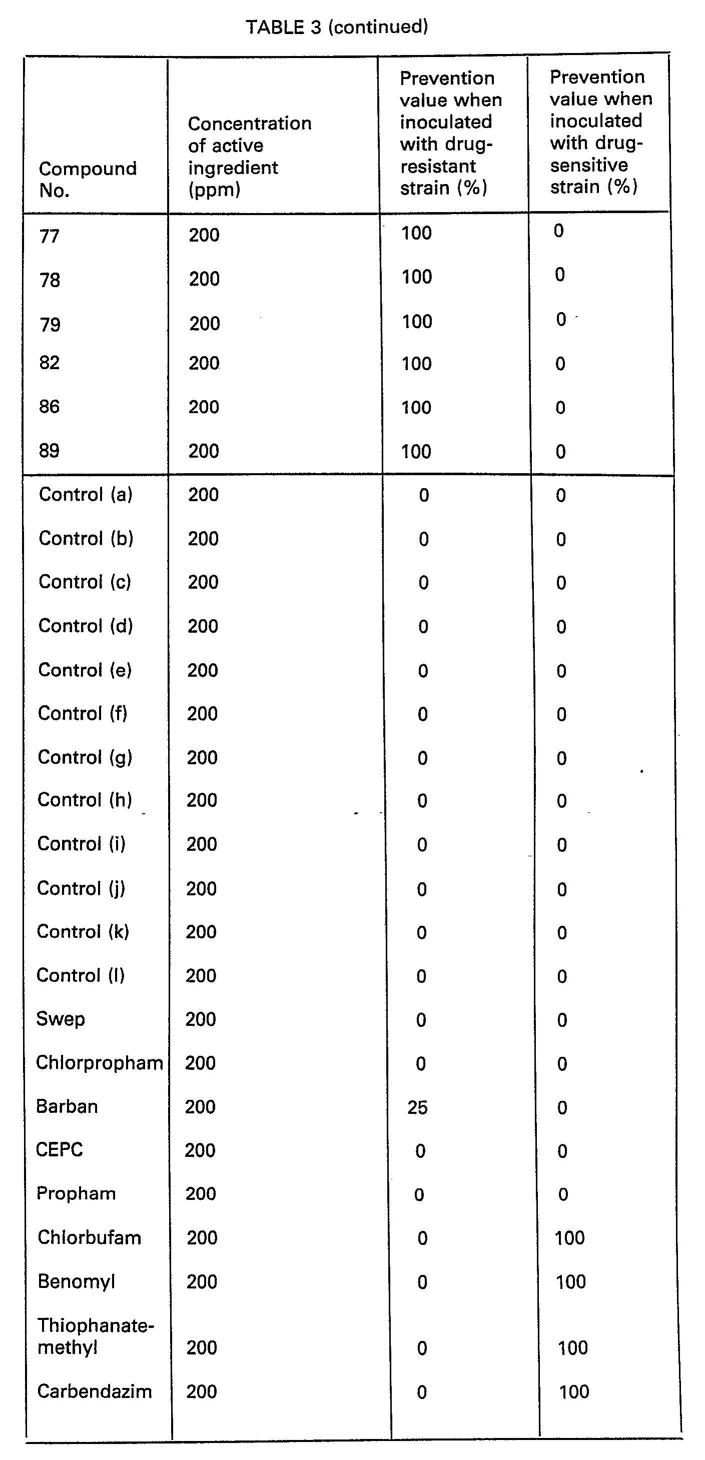

As understood from the results shown in Table 3, the N-phenylcarbamates (I) of the invention show an excellent preventive effect on the drug-resistant strain but do not show any preventive effect on the tested drug-sensitive strain. To the contrary, commercially available known fungicides such as Benomyl, Thiophanate-methyl and Carbendazim show a notable controlling effect on the drug-sensitive strain but not on the drug-resistant strain. Other tested compounds structurally similar to the N-phenylcarbamates (I) do not show any fungicidal activity on the drug-sensitive strain and the drug-resistant strain.

### Experiment 2

### Preventive effect on cercospora leaf spot of sugarbeet (Cercospora beticola):-

A flower pot of 90 ml volume was filled with sandy soil, and seeds of sugarbeet (var: Detroit dark red) were sowed therein. Cultivation was carried out in a greenhouse for 20 days. Onto the resulting seedlings, the test compound formulated in emulsifiable concentrate or wettable powder and diluted with water was sprayed at a rate of 10 ml per pot. Then, the seedlings were inoculated with a spore suspension of the drug-resistant or drug-sensitive strain of Cercospora beticola by spraying. The pot was covered with a polyvinyl chloride sheetto make a condition of high humidity, and cultivation was continued in the greenhouse for 10 days. The degree of damage was determined in the same manner as in Experiment 1, and the results are shown in Table 4.

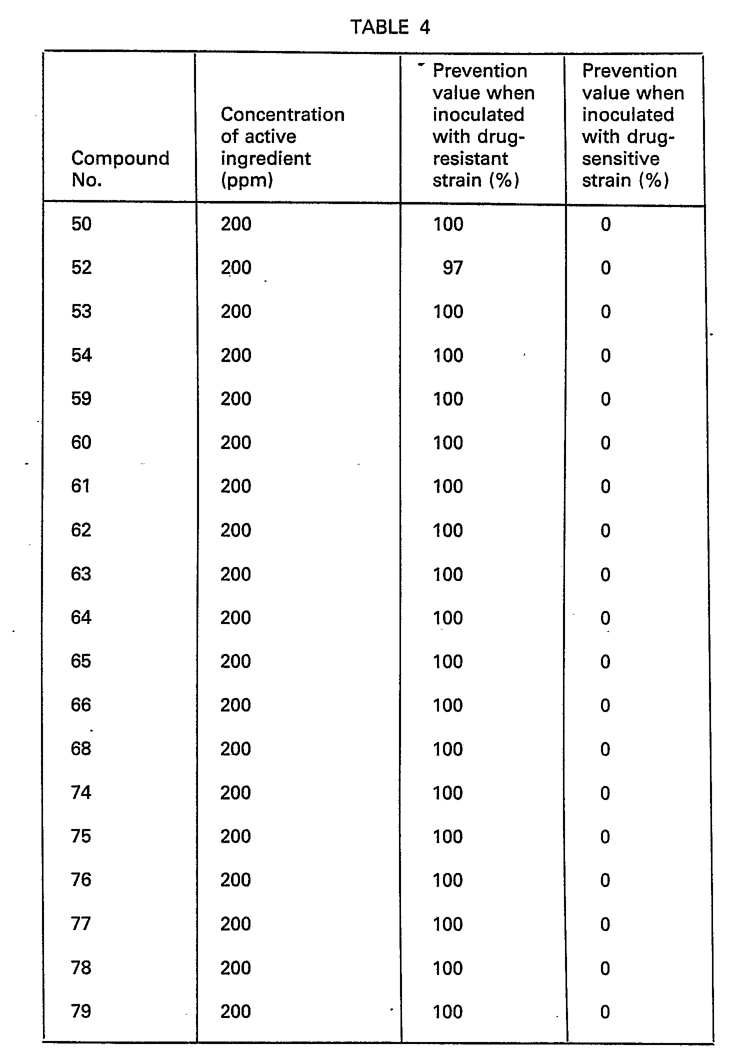

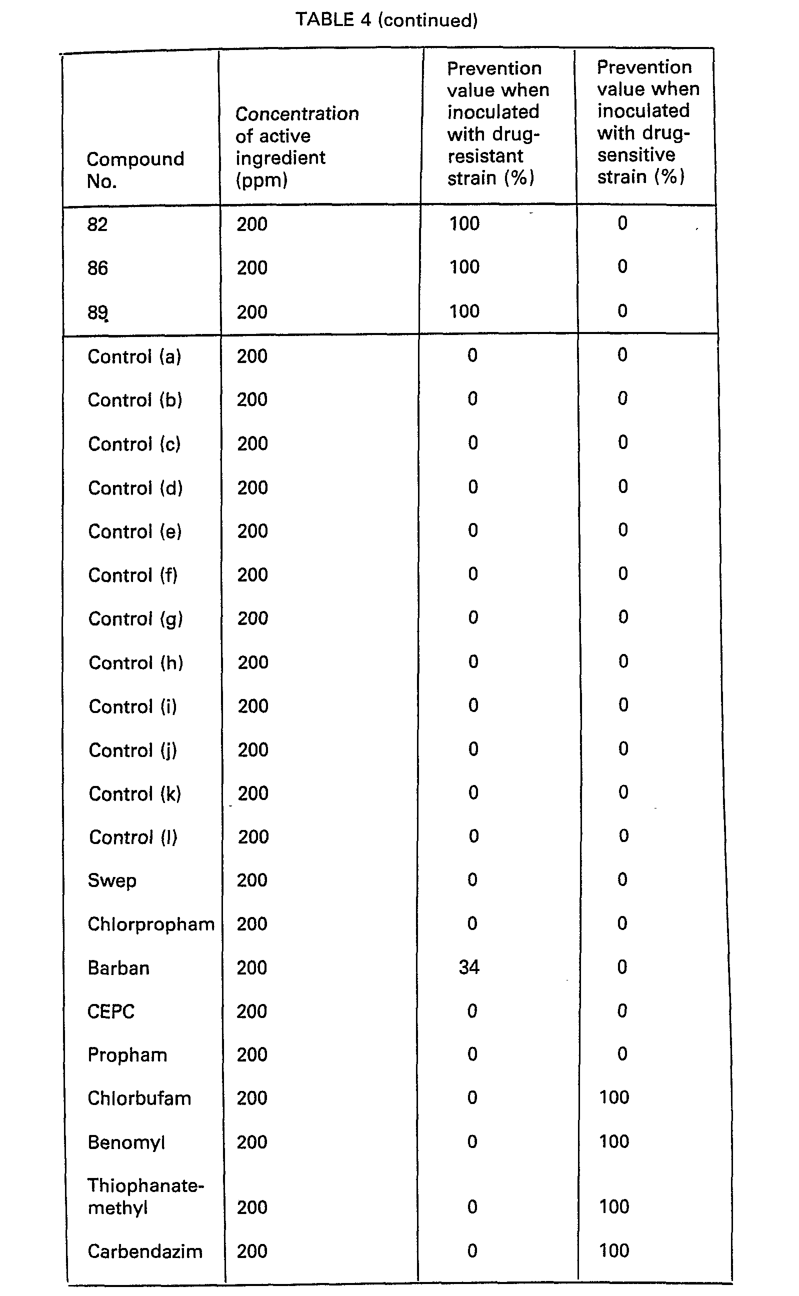

As understood from the results shown in Table 4, the N-phenylcarbamates (I) of the invention show an excellent preventive effect on the drug-resistant strain but do not shown any preventive effect on the tested drug-sensitive strain. To the contrary, commercially available known fungicides such as Benomyl and Thiophanate-methyl show a notable controlling effect on the drug-sensitive strain but not on the drug-resistant strain. Other tested compounds structurally similar to the N-phenylcarbamates (I) do not show any fungicidal activity on the drug-sensitive strain and the drug-resistant strain.

### Experiment 3

### Preventive effect on scab of pear (Venturia nashicola):-

A plastic pot of 90 ml volume was filled with sandy soil, and seeds of pear (var: Chojuro) were sowed therein. Cultivation was carried out in a greenhouse for 20 days. Onto the resulting seedlings, the test compound formulated in emulsifiable concentrate or wettable powder and diluted with water was sprayed at a rate of 10 ml per pot. Then, the seedlings were inoculated with a spore suspension of the drug-resistant or drug-sensitive strain of Venturia nashicola by spraying. The resulting plants were placed at 20°C under a condition of high humidity for 3 days and then at 20°C under irradiation with a fluorescent lamp for 20 days. The degree of damage was determined in the same manner as in Experiment 1, and the results are shown in Table 5.

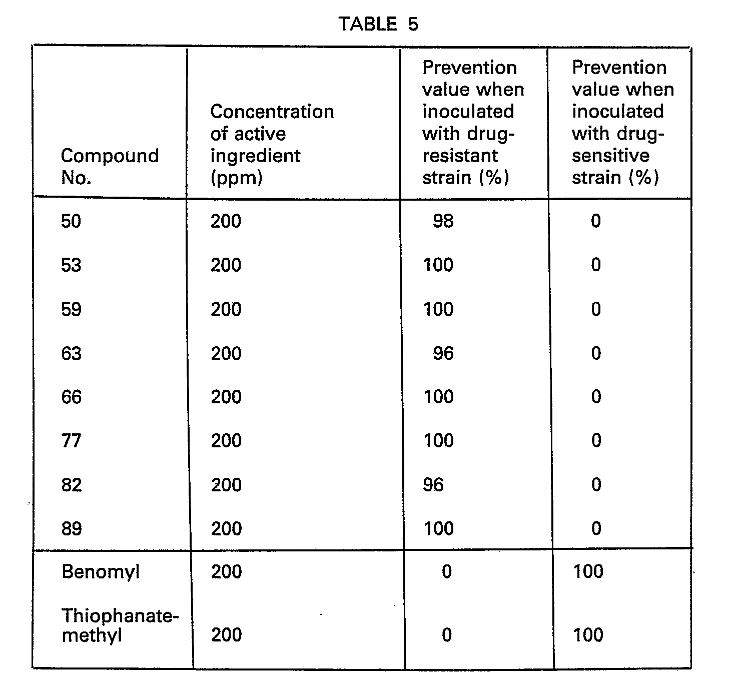

As understood from the results shown in Table 5, the N-phenylcarbamates (I) of the invention show an excellent preventive effect on the drug-resistant strain but do not show any preventive effect on the tested drug-sensitive strain. To the contrary, commercially available known fungicides such as Benomyl and Thiophanate-methyl show a notable controlling effect on the drug-sensitive strain but not on the drug-resistant strain.

### Experiment 4

### Preventive effect on gray mold of cucumber (Botrytis cinerea):-

Plastic pots of 90 ml volume were filled with sandy soil, and seeds of cucumber (var: Sagami-hanjiro) were sowed therein. Cultivation was carried out in a greenhouse for 8 days to obtain cucumber seedlings expanding cotyledons. Onto the resulting seedlings, the test compound formulated in emulsifiable concentrate or wettable powder and diluted with water was sprayed at a rate of 10 ml per pot. After airdrying, the seedlings were inoculated with mycelial disks (5 mm in diameter) of the drug-resistant or drug-sensitive strain of Botrytis cinerea by putting them on the leaf surfaces. After the plants were infected by incubating under high humidity at 20°C for 3 days, the rates of disease severity were observed. The degree of damage was determined in the same manner as in Experiment 1, and the results are shown in Table 6.

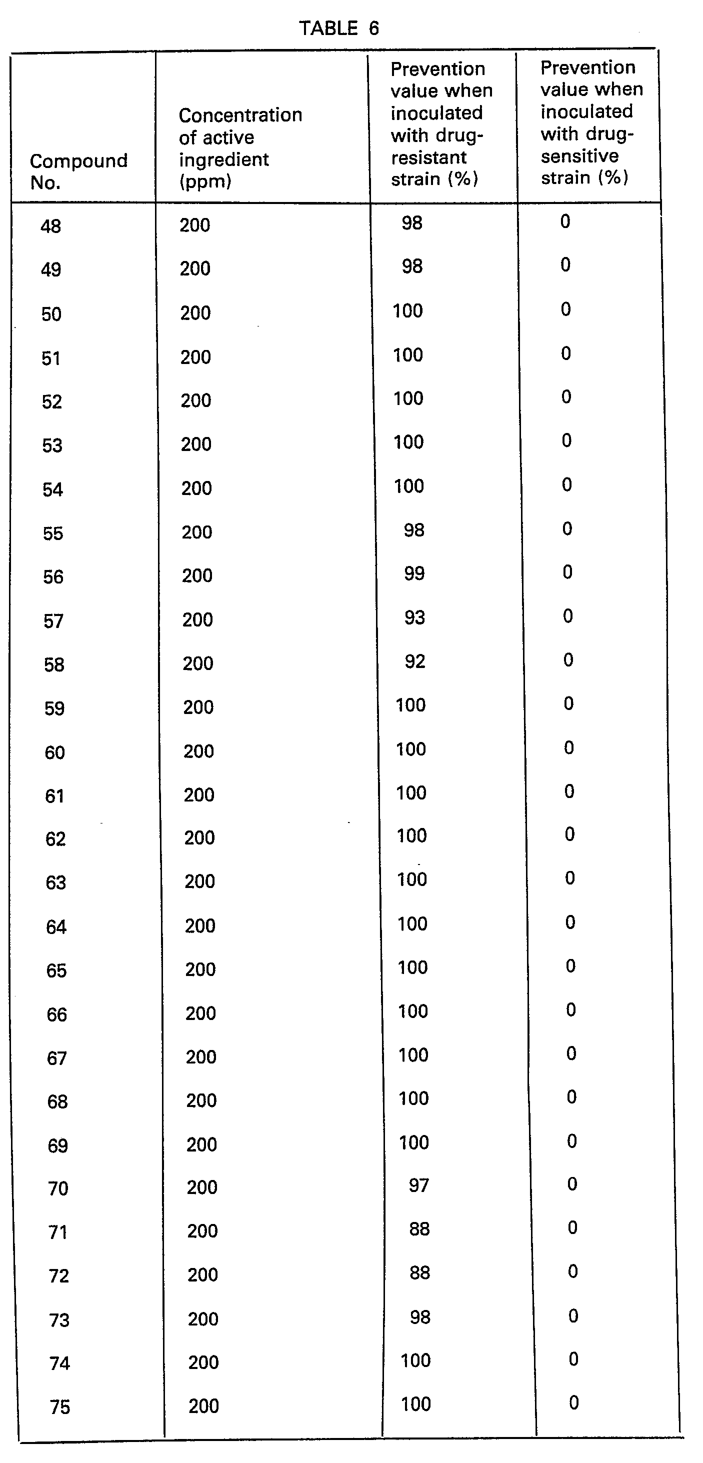

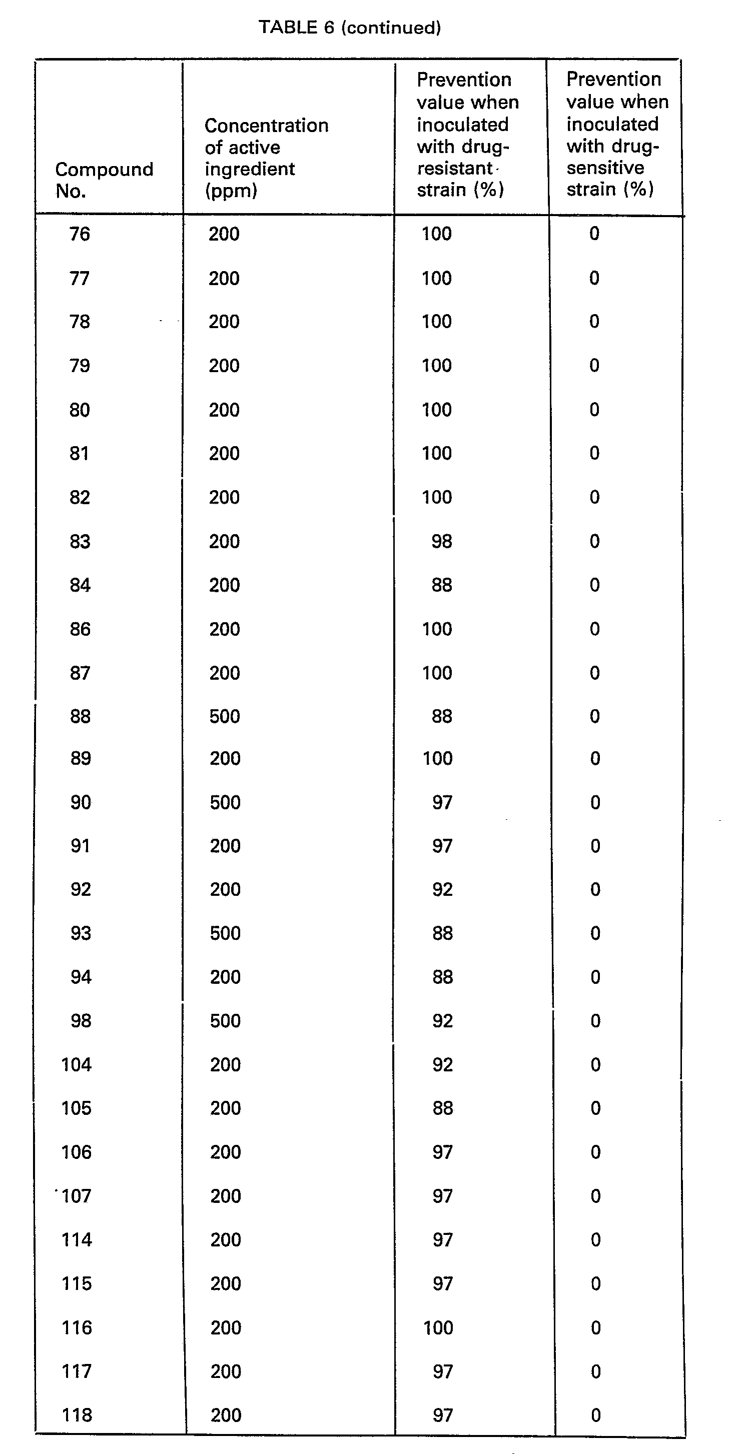

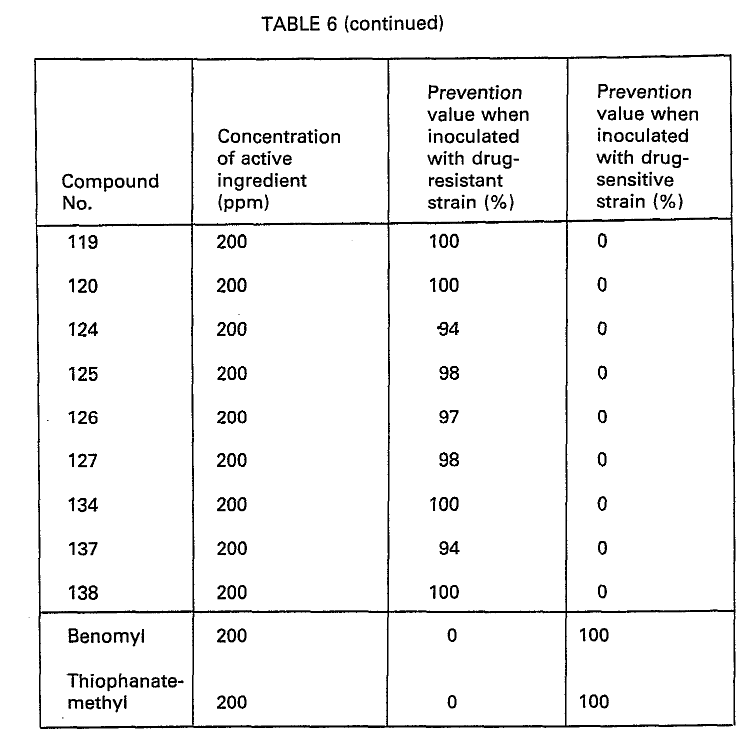

As understood from the results shown in Table 6, the N-phenylcarbamates (I) of the invention show an excellent preventive effect on the drug-resistant strain but do not show any preventive effect on the tested drug-sensitive strain. To the contrary, commercially available known fungicides such as Benomyl and Thiophanate-methyl show a notable controlling effect on the drug-sensitive strain but not on the drug-resistant strain.

### Experiment 5

### Preventive effect on powdery mildew of cucumber (Sphaerotheca fuliginea):-

A plastic pot of 90 ml volume was filled with sandy soil, and seeds of cucumber (var: Sagami-hanjiro) were sowed therein. Cultivation was carried out in a greenhouse for 8 days. Onto the resulting seedlings having cotyledons, the test compound(s) formulated in emulsifiable concentrate or wettable powder and diluted with water were sprayed at a rate of 10 ml per pot. Then, the seedlings were inoculated with a mixed spore suspension of the drug-resistant and drug-sensitive strain of Sphaerotheca fuliginea by spraying and further cultivated in the greenhouse. Ten days thereafter, the infectious state of the plants was observed. The degree of damage was determined in the same manner as in Experiment 1, and the results are shown in Table 7.

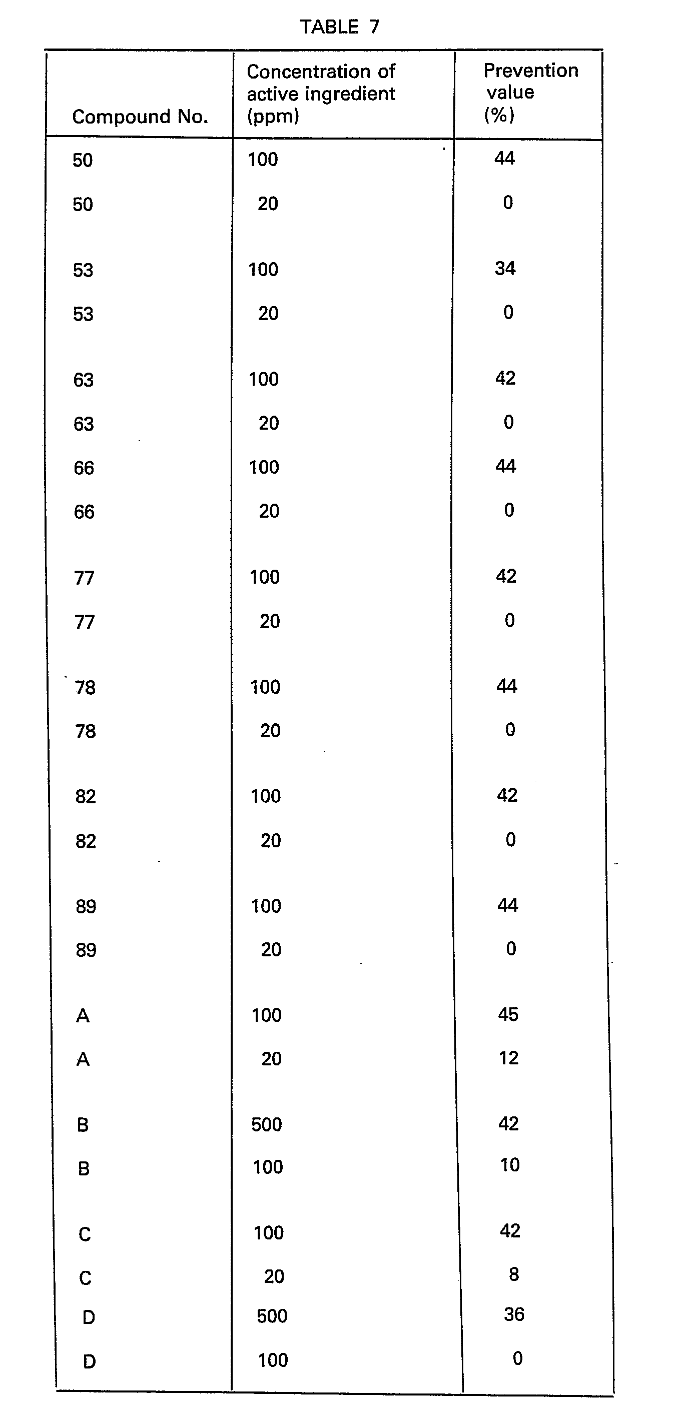

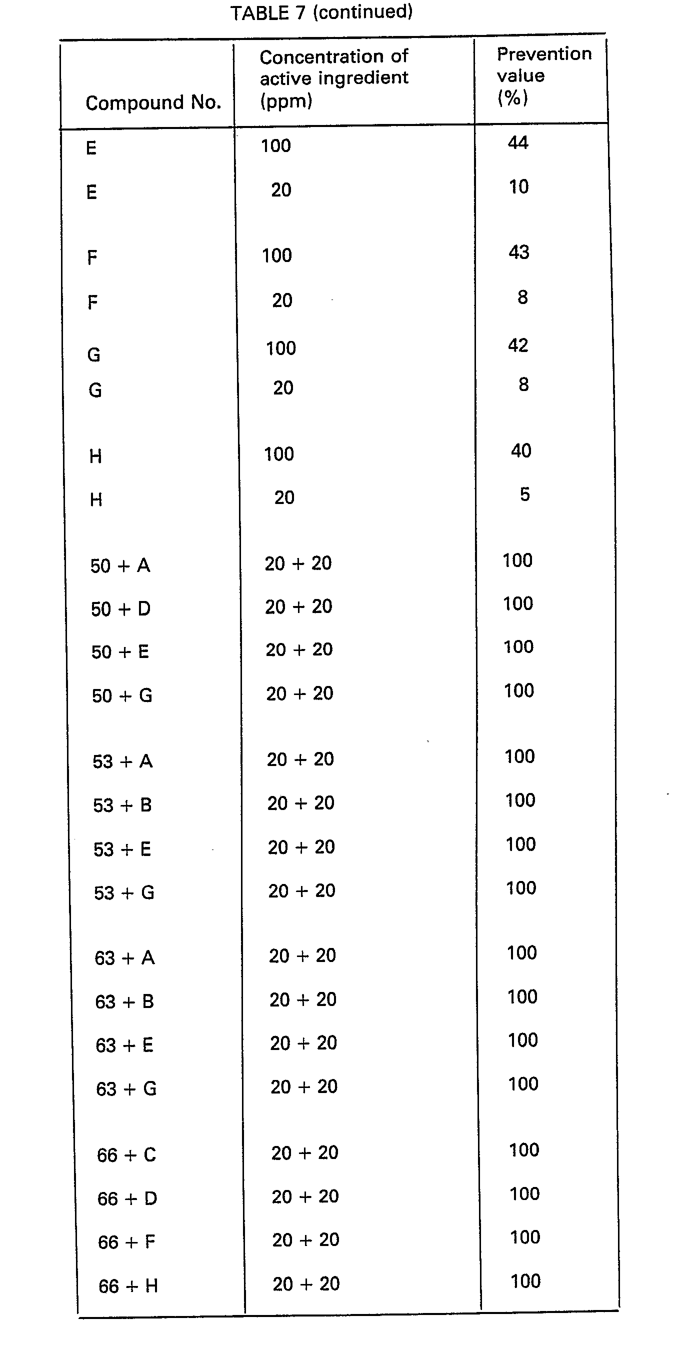

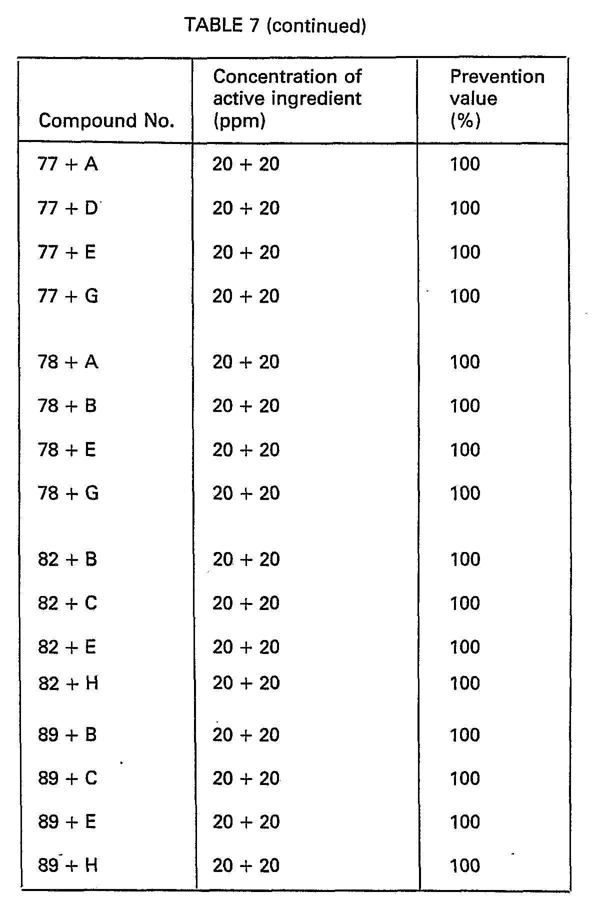

As understood from the results shown in Table 7, the combined use of the N-phenylcarbamates (I) of the invention with benzimidazole thiophanate and/or cyclic imide fungicides show much more excellent preventive effect than their sole use.

### Experiment 6

### Preventive effect on gray mold of tomato (Botrytis cinerea):-

A plastic pot of 90 ml volume was filled with sandy soil, and seeds of tomato (var: Fukuji No. 2) were sowed therein. Cultivation was carried out in a greenhouse for 4 weeks. Onto the resulting seedlings at the 4-leaf stage, the test compound(s) formulated in emulsifiable concentrate or wettable powder and diluted with water were sprayed at a rate of 10 ml per pot. Then, the seedlings were inoculated with a mixed spore suspension of the drug-resistant and drug-sensitive strain of Botrytis cinerea by spraying and placed at 20°C in a room of high humidity for 5 days. The degree of damage was determined in the same manner as in Experiment 1, and the results are shown in Table 8.

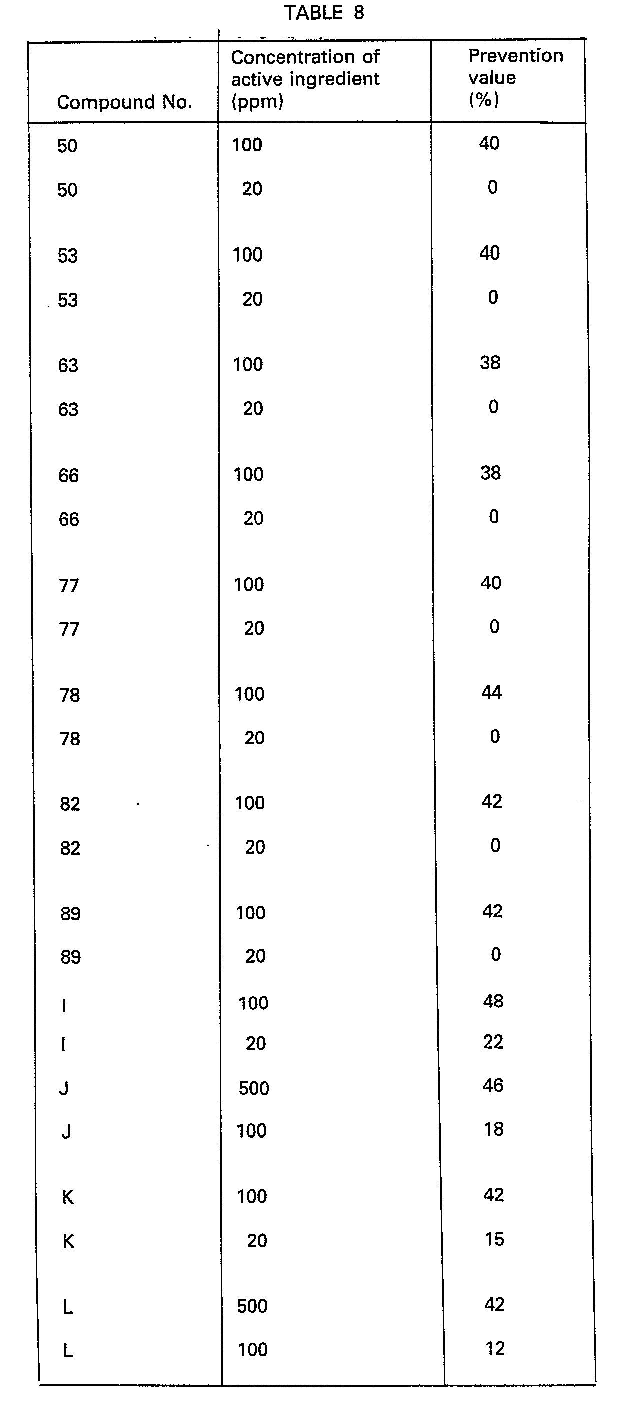

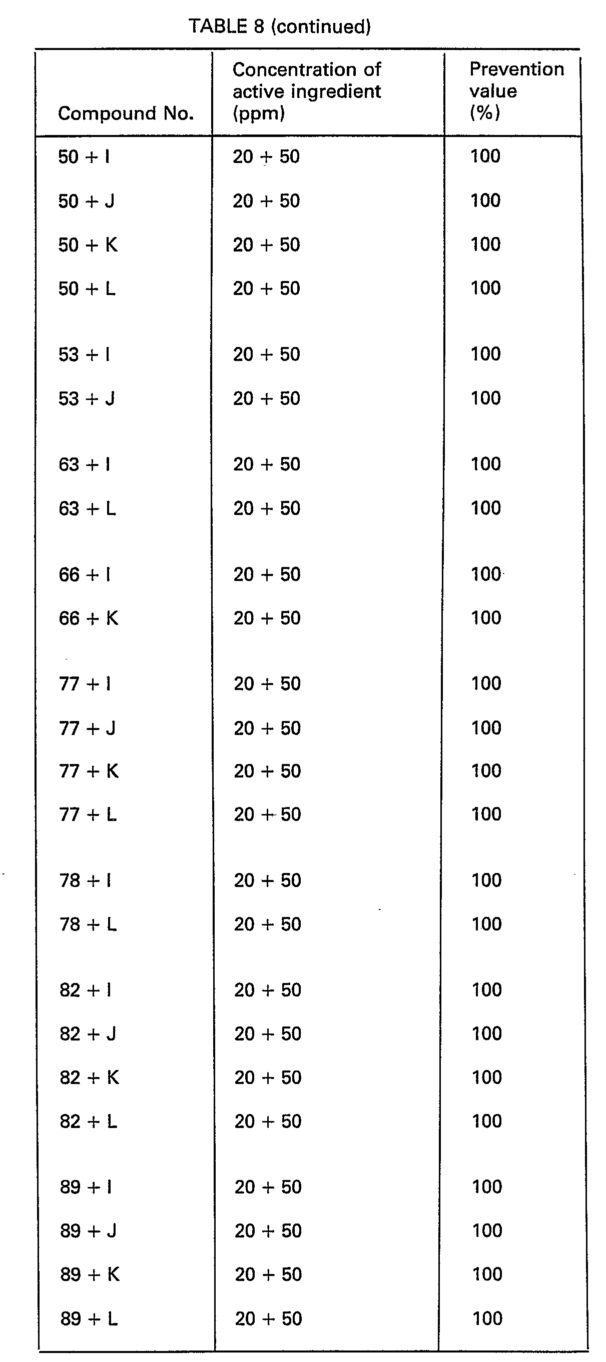

As understood from the results shown in Table 8, the combined use of the N-phenylcarbamates (I) of the invention with benzimidazole thiophanate and/or cyclic imide fungicides show much more excellent preventive effect than their sole use.

## Claims (Claims for the following Contracting State(s) : s: BE CH DE FR GB IT LI LU NL SE)

1. A fungicidal composition which comprises as an active ingredient a fungicidally effective amount of an N-phenylcarbamate of the formula: wherein R¹ is a C₂-C₄ alkyl group, a lower alkenyl group, a lower cyanoalkenyl group, a lower alkynyl group, a lower alkyl group substituted with at least one hydroxyl or cyano group, or a group of the formula: in which R⁴ is a lower alkyl group, a lower alkenyl group, a lower alkynyl group or a lower haloalkyl group, R⁵ is a hydrogen atom or a lower alkyl group and n is 2, 3 or 4; R² is a lower alkyl group, a lower alkenyl group, a lower alkynyl group or a lower alkyl group substituted with at least one halogen atom, lower alkoxy or lower cycloalkyl group; R³ is a C₁-C_{S} alkyl group, a C₃-C₈ alkenyl group, a C₃-Cₑ alkynyl group, a lower cycloalkyl group, a lower haloalkenyl group, a lower haloalkynyl group, a lower aralkyl group or a lower alkyl group substituted with at least one halogen atom, cyano, lower alkoxy, lower alkenyloxy, lower haloalkoxy, phenoxy, lower aralkyloxy or lower cycloalkyl group; X and Y are each independently an oxygen atom or a sulfur atom; and Z is a hydrogen atom, a lower alkyl group, a lower alkenyl group, a lower alkynyl group, a lower cycloalkyl group, a lower halo-alkenyl group, a lower alkyl group substituted with at least one halogen atom, cyano, lower alkoxy or lower alkoxycarbonyl group, or a group of the formula: -COR⁶ or -S0₂R⁶ in which R⁶ is a lower alkyl group, a lower alkenyl group, a lower alkynyl group, a lower cycloalkyl group, a lower haloalkenyl group, a lower alkyl group substituted with at least one halogen atom, cyano, lower alkoxy, lower cycloalkyl or phenoxy group (the said phenoxy group being optionally substituted with at least one halogen atom and/or at least one alkyl group), a phenyl group, a furyl group, a thienyl group, a phenyl group substituted with at least one halogen atom, cyano, nitro, trifluoromethyl, lower alkyl or lower alkoxy group, or an aralkyl group (the said aralkyl group being optionally substituted with at least one halogen and/or at least one alkyl group), together with an inert carrier or diluent.

2. A fungicidal composition as claimed in claim 1, which further comprises as an additional active ingredient at least one benzimidazole, thiophanate fungicide or cyclic imide fungicide.

3. A fungicidal composition as claimed in claim 2 wherein the benzimidazole fungicide is methyl 1-(butyl-carbamoyl)benzimidazol-2-ylcarbamate, 2-(2-furyl)benzimidazole, 2-(4-thiazolyl)benzimidazole or methyl benzimidazol-2-ylcarbamate.

4. A fungicidal composition as claimed in claim 2 wherein the thiophanate fungicide is 1,2-bis(3-methoxy-carbonyl-2-thioureido)benzene, 1,2-bis(3-ethoxycarbonyl-2-thioureido)benzene, 2-(O,S-dimethyl- phosphorylamino)-1-(3'-methoxycarbonyl-2'-thioureido)benzene or 2(0,0-dimethyl-thiophosphoryl- amino)-1-(3'-methoxycarbony)-2'-thioureido)-benzene.

5. A fungicidal composition as claimed in claim 2 wherein the cyclic imide fungicide is 3-(3',5'-dichlorophenyl)-1,2-dimethylcyclopropane-1,2-dicarboximide, 3-(3'-5'-dichlorophenyl)-I-isopropylcarbamoylimidazolidine-2,4-dione, 3-(3',5'-dichlorophenyl)-5-methyl-5-vinyloxazolidine-2,4-dione or ethyl (RS)-3(3',5'-dichlorophenyl)-5-methyl-2,4-dioxooxazolidine-5-carboxylate.

6. An N-phenylcarbamate of the formula: wherein R¹ is a C₂-C₄ alkyl group, a lower alkenyl group, a lower cyanoalkenyl group, a lower alkynyl group, a lower alkyl group substituted with at least one hydroxyl or cyano group, or a group of the formula 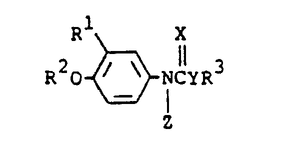 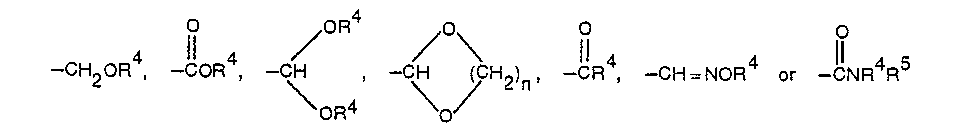 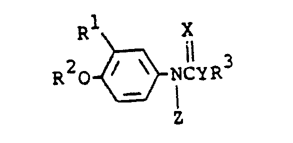 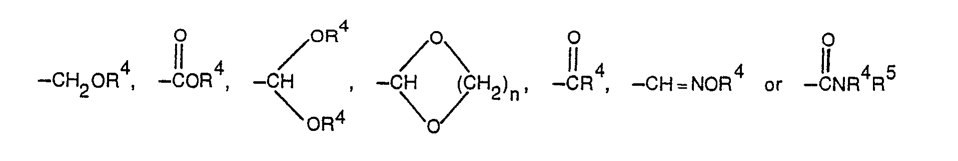 in which R⁴ is a lower alkyl group, a lower alkenyl group, a lower alkynyl group or a lower haloalkyl group, R⁵ is a hydrogen atom or a lower alkyl group and n is 2, 3 or 4; R² is a lower alkyl group, a lower alkenyl group, a lower alkynyl group or a lower alkyl group substituted with at least one halogen atom, lower alkoxy or lower cycloalkyl group; R³ is a C₁-C₈ alkyl group, a C₃-C₈ alkenyl group, a C3-Cs alkynyl group, a lower cycloalkyl group, a lower haloalkenyl group, a lower haloalkynyl group, a lower aralkyl group or a lower alkyl group substituted with at least one halogen atom, cyano, lower alkoxy, lower alkenyloxy, lower haloalkoxy, phenoxy, lower aralkyloxy or lower cycloalkyl group; X and Y are each independently an oxygen atom or a sulfur atom; and Z is a hydrogen atom, a lower alkyl group, a lower alkenyl group, a lower alkynyl group, a lower cycloalkyl group, a lower haloalkenyl group, a lower alkyl group substituted with at least one halogen atom, cyano, lower alkoxy or lower alkoxycarbonyl group, or a group of the formula: -COR⁶ or -S0₂R⁶ in which R⁶ is a lower alkyl group, a lower alkenyl group, a lower alkynyl group, a lower cycloalkyl group, a lower haloalkenyl group, a lower alkyl group substituted with at least one halogen atom, cyano, lower alkoxy, lower cycloalkyl or phenoxy group (the said phenoxy group being optionally substituted with at least one halogen and/or at least one alkyl group), a phenyl group, a furyl group, a thienyl group, a phenyl group substituted with at least one halogen atom, cyano, nitro, trifluoromethyl, lower alkyl or lower alkoxy group, or an aralkyl group (the said aralkyl group being optionally substituted with at least one halogen atom and/or at least one alkyl group).

7. An N-phenylcarbamate as claimed in claim 6 wherein R¹ is a C₂-C₄ alkyl group, a C3-C₄ alkenyl group, an ethynyl group, a cyanomethyl group, a methoxymethyl group, an ethoxymethyl group, a methoxycarbonyl group, an ethoxycarbonyl group, a dimethoxymethyl group, a methylenedioxymethyl group, an acetyl group, a propionyl group, a methoxy-iminomethyl group or an N-ethylcarbamoyl group; R² is a Cₗ-C₃ alkyl group, an allyl group, a propargyl group, a difluoromethyl group, a 2-chloroethyl group, a 2-fluoroethyl group, a 2,2,2-trifluoroethyl group, a 2-methoxyethyl group or a 2-cyclopropylethyl group; R³a is a C₁-C₈ alkyl group, a C₃-Cₛ alkenyl group, a C₃-Cg alkynyl group, a cyclobutyl group, a 4-chloro-2-butenyl group, a 4-chloro-2-butynyl group, a 1-phenylethyl group, a 2-fluoroethyl group, a 1-(chloromethyl)ethyl group, a 2-cyanoethyl group, a 1-(methoxymethyl)ethyl group, a 2-chloro-1-(methoxymethyl)-ethyl group, a 1-(allyloxymethyl)ethyl group, a 1-(2-chloro-ethoxymethyl)ethyl group, a 1-(phenoxymethyl)ethyl group, a 1-(benzyloxymethyl)ethyl group or a 1-(cyclopropyl)ethyl group; X and Y are each independently an oxygen atom or a sulfur atom; and Z is a hydrogen atom, a methyl group, an allyl group, a propargyl group, a cyanomethyl group, an ethoxycarbonylmethyl group, an acetyl group, a 1-methyl- butanoyl group, a cyclopropanecarbonyl group, a chloroacetyl group, a benzyl group, a 2-chlorobenzoyl group or a methylsulfonyl group.

8. An N-phenylcarbamate as claimed in claim 6 wherein R' is an ethyl group, a propyl group, an allyl group, a methoxymethyl group, an ethoxymethyl group or an ethoxycarbonyl group; R² is an ethyl group, a propyl group, an allyl group, a propargyl group or a 2-chloroethyl group; R³ is a methyl group, an ethyl group, an isopropyl group, a sec-butyl group, a 1-methyl-2-propenyl group, a 1-methyl-2-propynyl group, a 4-chloro-2-butynyl group, a 1-phenyl-ethyl group, a 2-fluoroethyl group, a 1-(chloromethyl)ethyl group, a 2-cyanoethyl group or a 1-(methoxymethyl)ethyl group; X is an oxygen atom; Y is an oxygen atom or a sulfur atom; and Z is a hydrogen atom, an acetyl group or a benzoyl group.

9. An N-phenylcarbamate which is isopropyl N-(3-ethyl-4-ethoxyphenyl)carbamate, isopropyl N-(3-propyl-4-ethoxyphenyl)carbamate, 1-methyl-2-propynyl N-(3-propyl-4-ethoxyphenyl)carbamate, isopropyl N-(3-methoxymethyl-4-ethoxyphenyl)carbamate, sec-butyl N-(3-methoxymethyl-4-ethoxyphenyl)carbamate, 1-(methoxymethyl)-ethyl N-(3-methoxymethyl-4-ethoxyphenyl)carbamate or isopropyl N-(3-ethoxymethyl-4-ethoxyphenyl)carbamate.

10. A method for controlling plant pathogenic fungi which comprises applying a fungicidally effective amount of at least one N-phenylcarbamate of the formula: 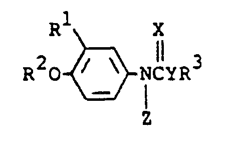 wherein R¹, R², R³, X, Y and Z are each as defined in claim 1, to plant pathogenic fungi.

11. A method as claimed in claim 10 wherein the plant pathogenic fungi is a drug-resistant strain.

12. A method for controlling plant pathogenic fungi which comprises applying a fungicidally effective amount of a mixture of an N-phenylcarbamate of the formula: 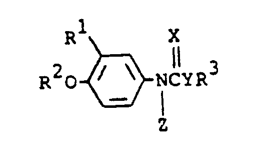 wherein R¹, R², R³, X, Y and Z are each as defined in claim 1 and a benzimidazole, thiophanate and/or a cyclic imide fungicide, to plant pathogenic fungi.

13. A process for producing an N-phenylcarbamate of the formula: 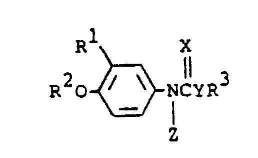 wherein R¹, R², R³, X, Y and Z are as defined in claim 6, which process comprises reacting an aniline of the formula: 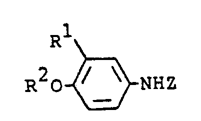 wherein R¹, R² and Z are each as defined above, with a chloroformate of the formula: 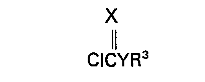 wherein R³, X and Y are as defined above.

14. A process for producing an N-phenylcarbamate of the formula: 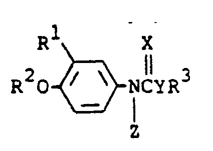 wherein Z is hydrogen and R¹, R², R³, X and Y are each as defined in claim 6, which process comprises reacting a phenyl isocyanate or isothiocyanate of the formula: 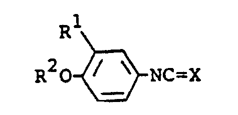 wherein R¹, R² and X are each as defined above, with an alcohol or thiol of the formula: 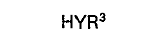 wherein R³ and Y are each as defined above.

15. A process for producing an N-phenylcarbamate of the formula: 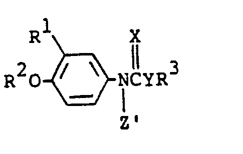 wherein R¹, R², R³, X and Y are each as defined in claim 6 and Z' is a lower alkyl group, a lower alkenyl group, a lower alkynyl group, a lower cycloalkyl group, a lower haloalkenyl group, a lower alkyl group substituted with at least one halogen atom, cyano, lower alkoxy or lower alkoxy-carbonyl group, or a group of the formula: -COR⁶ or-S0₂R⁶ in which R⁶ is a lower alkyl group, a lower alkenyl group, a lower alkynyl group, a lower cycloalkyl group, a lower haloalkenyl group, a lower alkyl group substituted with at least one halogen atom, cyano, lower alkoxy, lower cycloalkyl or phenoxy group, (the said phenoxy group being optionally substituted with at least one halogen atom and/or at least one alkyl group), a phenyl group, a furyl group, a thienyl group, a phenyl group substituted with at least one halogen atom, cyano, nitro, trifluoromethyl, lower alkyl or lower alkoxy group, or an aralkyl group (the said aralkyl group being optionally substituted with at least one halogen atom and/or at least one alkyl group), which comprises reacting an N-phenylcarbamate of the formula 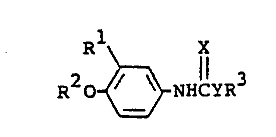 wherein R¹, R², R³, X and Y are each as defined above, with a halide of the formula: 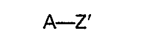 wherein A is a halogen atom and Z' is as defined above.

## Claims (Claims for the following Contracting State(s) : AT)

1. A process for producing an N-phenylcarbamate of the formula: 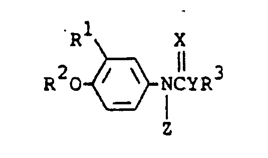 wherein R¹ is a Cg-C₄ alkyl group, a lower alkenyl group, a lower cyanoalkenyl group, a lower alkynyl group, a lower alkyl group substituted with at least one hydroxyl or cyano group, or a group of the formula: 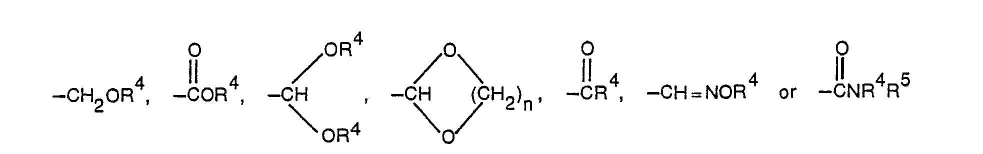 in which R⁴ is a lower alkyl group, a lower alkenyl group, a lower alkynyl group or a lower haloalkyl group, R⁵ is a hydrogen atom or a lower alkyl group and n is 2, 3 or 4; R² is a lower alkyl group; a lower alkenyl group, a lower alkynyl group or a lower alkyl group substituted with at least one halogen atom, lower alkoxy or lower cycloalkyl group; R³ is a C₁-C₈ alkyl group, a C₃-Cₑ alkenyl group, a C₃-C₈ alkynyl group, a lower cycloalkyl group, a lower haloalkenyl group, a lower haloalkynyl group, a lower aralkyl group or a lower alkyl group substituted with at least one halogen atom, cyano, lower alkoxy, lower alkenyloxy, lower haloalkoxy, phenoxy, lower aralkyloxy or lower cycloalkyl group; X and Y are each independently an oxygen atom or a sulfur atom; and Z is a hydrogen atom, a lower alkyl group, a lower alkenyl group, a lower alkynyl group, a lower cycloalkyl group, a lower haloalkenyl group, a lower alkyl group substituted with at least one halogen atom, cyano, lower alkoxy or lower alkoxycarbonyl group, or a group of the formula: -COR⁶ or -SO₂R⁶ in which R⁶ is a lower alkyl group, a lower alkenyl group, a lower alkynyl group, a lower cycloalkyl group, a lower haloalkenyl group, a lower alkyl group substituted with at least one halogen atom, cyano, lower alkoxy, lower cycloalkyl or phenoxy group (the phenoxy group being optionally substituted with at least one halogen atom and/or at least one alkyl group), a phenyl group, a furyl group, a thienyl group, a phenyl group substituted with at least one halogen, cyano, nitro, trifluoromethyl, lower alkyl or lower alkoxy group, or an aralkyl group (the said aralkyl group being optionally substituted with at least one halogen atom and/or at least one alkyl group), which comprises reacting an aniline of the formula: 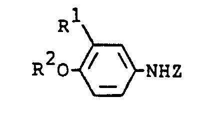 wherein R¹, R² and Z are each as defined above, with a chloroformate of the formula: 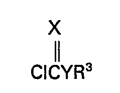 wherein R³, X and Y are each as defined above.

2. A process for producing an N-phenylcarbamate of the formula: 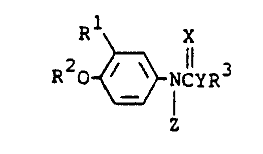 wherein Z is hydrogen and R¹, R², R³, X and Y are each as defined in claim 1, which comprises reacting a phenyl isocyanate or isothiocyanate of the formula: 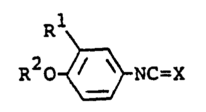 wherein R¹, R² and X are each as defined above, with an alcohol or thiol of the formula: 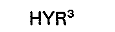 wherein R³a and Y are each as defined above.

3. A process for producing an N-phenylcarbamate of the formula 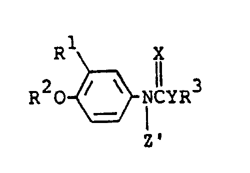 wherein R¹, R², R³, X and Y are each as defined in claim 1 and Z' is a lower alkyl group, a lower alkenyl group, a lower alkynyl group, a lower cycloalkyl group, a lower haloalkenyl group, a lower alkyl group substituted with at least one halogen atom, cyano, lower alkoxy or lower alkoxycarbonyl group, or a group of the formula: ―COR⁶ or―SO₂R⁶ in which R⁶ is a lower alkyl group, a lower alkenyl group, a lower alkynyl group, a lower cycloalkyl group, a lower haloalkenyl group, a lower alkyl group substituted with at least one halogen atom, cyano, lower alkoxy, lower cycloalkyl or phenoxy group (the said phenoxy group being optionally substituted with at least one halogen atom and/or at least one alkyl group; a phenyl group, a furyl group, a thenyl group, a phenyl group substituted with at least one halogen atom, cyano, nitro, trifluoromethyl, lower alkyl or lower alkoxy group, or an aralkyl group (the said aralkyl group being optionally substituted with at least one halogen atom and/or at least one alkyl group), which process comprises reacting an N-phenylcarbamate of the formula: 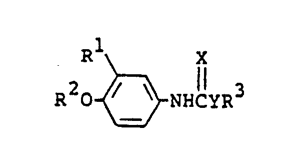 wherein R¹, R², R³, X and Y are each as defined above, with a halide of the formula: 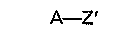 wherein A is a halogen atom and Z' is as defined above.

4. A method for controlling plant pathogenic fungi which comprises applying a fungicidally effective amount of at least one of the N-phenylcarbamates of the formula: 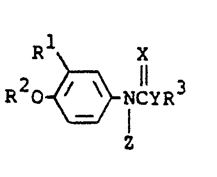 wherein R¹ is a C₂-C₄ alkyl group, a lower alkenyl group, a lower cyanoalkenyl group, a lower alkynyl group, a lower alkyl group substituted with at least one hydroxyl or cyano group, or a group of the formula: 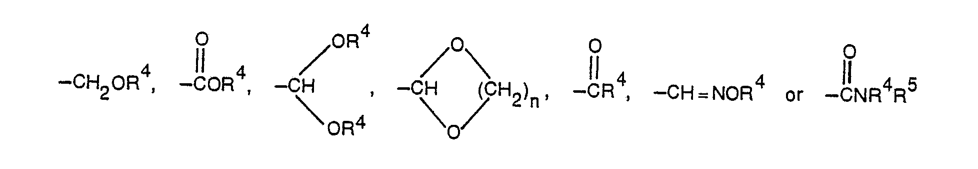 in which R⁴ is a lower alkyl group, a lower alkenyl group, a lower alkynyl group or a lower haloalkyl group, R⁵ is a hydrogen atom or a lower alkyl group and n is 2, 3 or 4; R² is a lower alkyl group, a lower alkenyl group, a lower alkynyl group or a lower alkyl group substituted with at least one halogen atom, lower alkoxy or lower cycloalkyl group; R³ is a C₁―C₈ alkyl group, a C₃-C₈ alkenyl group, a C₃-Cₑ alkynyl group, a lower cycloalkyl group, a lower haloalkenyl group, a lower haloalkynyl group, a lower aralkyl group or a lower alkyl group substituted with at least one halogen atom, cyano, lower alkoxy, lower alkenyloxy, lower haloalkoxy, phenoxy, lower aralkyloxy or lower cycloalkyl group; X and Y are each independently an oxygen atom or a sulfur atom; and Z is a hydrogen atom, a lower alkyl group, a lower alkenyl group, a lower alkynyl group, a lower cycloalkyl group, a lower haloalkenyl group, a lower alkyl group substituted with at least one halogen atom, cyano, lower alkoxy or lower alkoxycarbonyl group, or a group of the formula: -COR⁶ or -S0₂R⁶ in which R⁶ is a lower alkyl group, a lower alkenyl group, a lower alkynyl group, a lower cycloalkyl group, a lower haloalkenyl group, a lower alkyl group substituted with at least one halogen atom, cyano, lower alkoxy, lower cycloalkyl or phenoxy group (the said phenoxy group being optionally substituted with at least one halogen atom and/or at least one alkyl group), a phenyl group, a furyl group, a thienyl group, a phenyl group substituted with at least one halogen atom, cyano, nitro, trifluoromethyl, lower alkyl or lower alkoxy group, or an aralkyl group (the said aralkyl group being optionally substituted with at least one halogen and/or at least one alkyl group) to plant pathogenic fungi.

5. A method as claimed in claim 4 wherein the plant pathogenic fungi is a drug-resistant strain.

6. A method for controlling plant pathogenic fungi which comprises applying a fungicidally effective amount of a mixture of an N-phenylcarbamate of the formula: 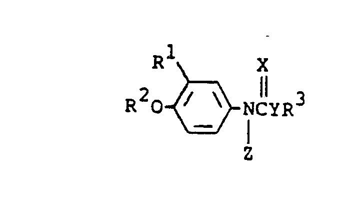 wherein R¹, R², R³, X, Y and Z are each as defined in claim 1 and a benzimidazole, thiophanate and/or a cyclic imide fungicide.

## Patentansprüche (Patentansprüche für folgende(n) Vertragsstaat(en) : BE CH DE FR GB IT LI LU NL SE)

1. Fungizides Mittel, das als wirksamen Bestandsteil eine fungizid wirksame Menge eines N-Phenylcarbamates der Formel: 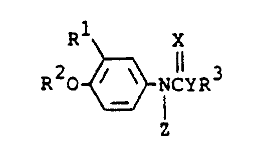 worin R¹ eine Alkylgruppe mit 2 bis 4 Kohlenstoffatomen, eine niedere Alkenylgruppe, eine niedere Cyanoalkenylgruppe, eine niedere Alkinylgruppe, eine niedere Alkylgruppe, die durch mindestens eine Hydroxyl-oder Cyanogruppe substituiert ist, oder eine Gruppe der Formel: 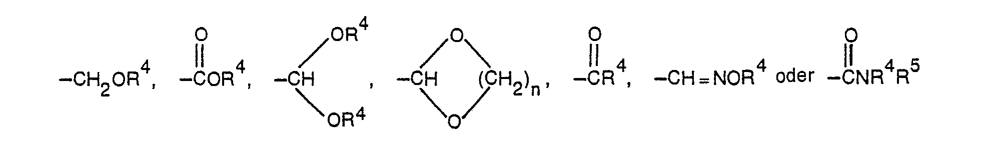 bedeutet, worin R' eine niedere Alkylgruppe, eine niedere Alkenylgruppe, eine niedere Alkinylgruppe oder eine niedere Halogenalkylgruppe ist, R⁵ ein Wasserstoffatom oder eine niedere Alkylgruppe ist und n für 2, 3 oder 4 steht; R² eine niedere Alkylgruppe, eine niedere Alkenylgruppe, eine niedere Alkinylgruppe oder eine niedere Alkylgruppe, die durch mindestens ein Halogenatom, mindestens eine niedere Alkoxygruppe oder mindestens eine niedere Cycloalkylgruppe substituiert ist, bedeutet; R³ eine Alkylgruppe mit 1 bis 8 Kohlenstoffatomen, eine Alkenylgruppe mit 3 bis 8 Kohlenstoffatomen, eine Alkinylgruppe mit 3 bis 8 Kohlenstoffatomen, eine niedere Cycloalkylgruppe, eine niedere Halogenalkenylgruppe, eine niedere Halogenalkinylgruppe, eine niedere Aralkylgruppe oder eine niedere Alkylgruppe, die durch mindestens ein Halogenatom oder mindestens eine Cyano-, niedere Alkoxy-, niedere Alkenyloxy-, niedere Halogenalkoxy, Phenoxy-, niedere Aralkyloxy- oder niedere Cycloalkylgruppe substituiert ist, bedeutet; X und Y jeweils unabhängig voneinander ein Sauerstoffatom oder ein Schwefelatom bedeuten; und Z ein Wasserstoffatom, eine niedere Alkylgruppe, eine niedere Alkenylgruppe, eine niedere Alkinylgruppe, eine niedere Cycloalkylgruppe, eine niedere Halogenalkenylgruppe, eine niedere Alkylgruppe, die durch mindestens ein Halogenatom oder mindestens eine Cyano-, niedere Alkoxy- oder niedere Alkoxycarbonyl-gruppe substituiert ist, oder eine Gruppe der Formel: 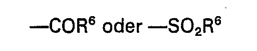 bedeutet, worin R⁶ eine niedere Alkylgruppe, eine niedere Alkenylgruppe, eine niedere Alkinylgruppe, eine niedere Cycloalkylgruppe, eine niedere Halogenalkenylgruppe, eine niedere Alkylgruppe, die durch mindestens ein Halogenatom oder mindestens eine Cyano-, niedere Alkoxy, niedere Cycloalkyl- oder Phenoxygruppe substituiert ist (wobei die genannte Phenoxygruppe gegenbenenfalls durch mindestens ein Halogenatom und/oder mindestens eine Alkylgruppe substituiert ist), eine Phenylgruppe, eine Furylgruppe, eine Thienylgruppe, eine Phenylgruppe, die durch mindestens ein Halogenatom oder mindestens eine Cyano-, Nitro-, Trifluormethyl-, niedere Alkyl- oder niedere Alkoxygruppe substituiert ist, oder eine Aralkylgruppe bedeutet (wobei die genannte Aralkylgruppe gegebenenfalls durch mindestens ein Halogenatom und/oder mindestens eine Alkylgruppe substituiert ist), zusammen mit einem inerten Träger oder Verdünnungsmittel enthält.

2. Fungizides Mittel nach Anspruch 1, dadurch gekennzeichnet, dass es ferner als zusätzlichen wirksamen Bestandteil mindestens ein fungizides Benzimidazol, fungizides Thiophanat oder fungizides cyclisches Imid enthält.

3. Fungizides Mittel nach Anspruch 2, dadurch gekennzeichnet, dass das fungizide Benzimidazol Methyl-1-(butylcarbamoyl)-benzimidazol-2-ylcarbamat, 2-(2-Furyl)-benzimidazol, 2-(4-Thiazolyl)-benz- imidiazol oder Methylbenzimidazol-2-ylcarbamat ist.

4. Fungizides Mittel nach Anspruch 2, dadurch gekennzeichnet, dass das fungizide Thiophanat 1,2 - Bis - (3 - methoxycarbonyl - 2 - thioureido) - benzol, 1,2 - Bis - (3 - ethoxycarbonyl - 2 - thioureido) - benzol, 2 - (O,S - Dimethyl - phosphorylamino) - 1 - (3' - methoxycarbonyl - 2' - thioureido) - benzol oder 2 - (0,0 - Dimethylthiophosphorylamino) - 1 - (3' - methoxycarbonyl - 2' - thioureido) - benzol ist.

5. Fungizides Mittel nach Anspruch 2, dadurch gekennzeichnet, dass das fungizide cyclische Imid 3 - (3',5' - Dichlorphenyl) - 1,2 - dimethylcyclopropan - 1,2 - dicarboximid, 3 - (3',5' - Dichlorphenyl) - 1 - isopropylcarbamoyi - imidazolidin - 2,4 - dion, 3 - (3',5' - Dichlorphenyl) - 5 - methyl - 5 - vinyloxazolidin - 2,4 - dion oder Ethyl - (R,S) - 3 - (3',5' - dichlorphenyl) - 5 - methyl - 2,4 - dioxooxazolidin - 5 - carboxylat ist.

6. N-Phenylcarbamat der Formel: 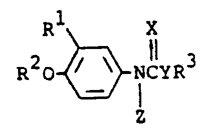 worin R¹ eine Alkylgruppe mit 2 bis 4 Kohlenstoffatomen, eine niedere Alkenylgruppe, eine niedere Cyanoalkenylgruppe, eine niedere Alkinylgruppe, eine niedere Alkylgruppe, die durch mindestens eine Hydroxyl-oder Cyanogruppe substituiert ist, oder eine Gruppe der Formel: 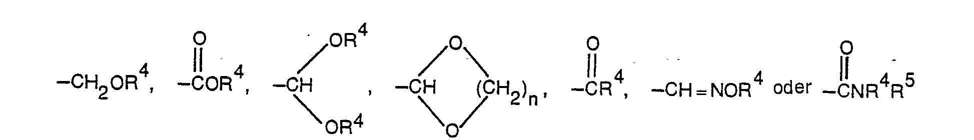 bedeutet, worin R⁴ eine niedere Alkylgruppe, eine niedere Alkenylgruppe, eine niedere Alkinylgruppe oder eine niedere Halogenalkylgruppe bedeutet, R⁵ ein Wasserstoffatom oder eine niedere Alkylgruppe bedeutet und n für 2, oder 4 steht; R² eine niedere Alkylgruppe, eine niedere Alkenylgruppe, eine niedere Alkinylgruppe oder eine niedere Alkylgruppe, die durch mindestens ein Halogenatom oder mindestens eine niedere Alkoxy- oder niedere Cycloalkylgruppe substituiert ist, bedeutet; R³ eine Alkylgruppe mit 1 bis 8 Kohlenstoffatomen, eine Alkenylgruppe mit 3 bis 8 Kohlenstoffatomen, eine Alkinylgruppe mit 3 bis 8 Kohlenstoffatomen, eine niedere Cycloalkylgruppe, eine niedere Halogenalkenylgruppe, eine niedere Halogenalkinylgruppe, eine niedere Aralkylgruppe oder eine niedere Alkylgruppe, die durch mindestens ein Halogenatom oder mindestens eine Cyano-, niedere Alkoxy₋, niedere Alkenyloxy-, niedere Halogenalkoxy-, Phenoxy-, niedere Aralkyloxy- oder niedere Cycloalkylgruppe substituiert ist, bedeutet; X und Y jeweils unabhängig voneinander ein Sauerstoffatomen oder ein Schwefelatom bedeuten; und Z ein Wasserstoffatom, eine niedere Alkylgruppe, eine niedere Alkenylgruppe, eine niedere Alkinylgruppe, eine niedere Cycloalkylgruppe, eine niedere Halogenalkenylgruppe, eine niedere Alkylgruppe, die durch mindestens ein Halogenatom oder mindestens eine Cyano-, niedere Alkoxy- oder niedere Alkoxycarbonylgruppe substituiert ist, oder eine Gruppe der Formel: 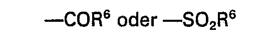 bedeutet, worin R⁶ eine niedere Alkylgruppe, eine niedere Alkenylgruppe, eine niedere Alkinylgruppe, eine niedere Cycloalkylgruppe, eine niedere Halogenalkenylgruppe, eine niedere Alkylgruppe, die durch mindestens ein Halogenatom oder mindestens eine Cyano-, niedere Alkoxy-, niedere Cycloalkyl- oder Phenoxygruppe substituiert ist (wobei die genannte Phenoxygruppe gegenbenenfalls durch mindestens ein Halogenatom und/oder mindestens eine Alkylgruppe substituiert ist), eine Phenylgruppe, eine Furylgruppe, eine Thienylgruppe, eine Phenylgruppe, die durch mindestens ein Halogenatom oder mindestens eine Cyano-, Nitro-, Trifluormethyl-, niedere Alkyl- oder niedere Alkoxygruppe substituiert ist, oder eine Aralkylgruppe bedeutet (wobei die genannte Aralkylgruppe gegebenenfalls durch mindestens ein Halogenatom und/oder mindestens eine Alkylgruppe substituiert ist).

7. N-Phenylcarbamat nach Anspruch 6, worin R¹ eine Alkylgruppe mit 2 bis 4 Kohlenstoffatomen, eine Alkenylgruppe mit 3 oder 4 Kohlenstoffatomen, eine Ethinylgruppe, eine Cyanomethylgruppe, eine Methoxymethylgruppe, eine Ethoxymethylgruppe, eine Methoxycarbonylgruppe, eine Ethoxymethylgruppe, eine Dimethoxymethylgruppe, eine Methylendioxymethylgruppe, eine Acetylgruppe, eine Propionylgruppe, eine Methoxyiminomethylgruppe oder eine N-Ethylcarbamoylgruppe bedeutet; R² eine Alkylgruppe mit 1 bis 3 Kohlenstoffatomen, eine Allylgruppe, eine Propargylgruppe, eine Difluormethylgruppe, eine 2-Chlorethylgruppe, eine 2-Fluorethylgruppe, eine 2,2,2-Trifluorethylgruppe, eine 2-Methoxyethylgruppe oder eine 2-Cyclopropylethylgruppe bedeutet; R³ eine Alkylgruppe mit 1 bis 8 Kohlenstoffatomen, eine Alkenylgruppe mit 3 bis 8 Kohlenstoffatomen, eine Alkinylgruppe mit 3 bis 8 Kohlenstoffatomen, eine Cyclobutylgruppe, eine 4-Chlor-2-butenylgruppe, eine 4-Chlor-2-butinylgruppe, eine 1-Phenylethylgruppe, eine 2-Fluorethylgruppe, eine 1-(Chlormethyl)-ethylgruppe, eine 2-Cyanoethylgruppe, eine 1-(Methoxymethyl)-ethylgruppe, eine 2-Chlor-1-(methoxymethyl)-ethylgruppe, eine 1-(Allyloxymethyl)-ethylgruppe, eine 1-(2-Chlorethoxymethyl)-ethylgruppe, eine 1-(Phenoxymethyl)-ethylgruppe, eine 1-(Benzyloxymethyl)-ethylgruppe oder eine 1-(Cyclopropyl)-ethylgruppe bedeutet; X und Y jeweils unabhängig voneinander ein Sauerstoffatom oder ein Schwefelatom bedeuten; und Z ein Wasserstoffatom, eine Methylgruppe, eine Allylgruppe, eine Propargylgruppe, eine Cyanomethylgruppe, eine Ethoxycarbonylmethylgruppe, eine Acetylgruppe, eine 1-Methylbutanoylgruppe, eine Cyclopropancarbonylgruppe, eine Chloracetylgruppe, eine Benzolylgruppe, eine 2-Chlorbenzoylgruppe oder eine Methylsulfonylgruppe bedeutet.

8. N-Phenylcarbamat nach Anspruch 6, worin R¹ eine Ethylgruppe, eine Propylgruppe, eine Allylgruppe, eine Methoxymethylgruppe, eine Ethoxymethylgruppe oder eine Ethoxycarbonylgruppe bedeutet; R² eine Ethylgruppe, eine Propylgruppe, eine Allylgruppe, eine Propargylgruppe oder eine 2-Chlorethylgruppe bedeutet; R³ eine Methylgruppe, eine Ethylgruppe, eine Isopropylgruppe, eine sek.-Butylgruppe, eine 1-Methyl-2-propenylgruppe, eine 1-Methyl-2-propinylgruppe, eine 4-Chlor-2-butinylgruppe, eine 1-Phenylethylgruppe, eine 2-Fluorethylgruppe, eine 1-(Chlormethyl)-ethylgruppe, eine 2-Cyanoethylgruppe oder eine 1-(Methoxymethyl)-ethylgruppe bedeutet; X ein Sauerstoffatom bedeutet; Y ein Sauerstoffatom oder ein Schwefelatom bedeutet; und Z ein Wasserstoffatom, eine Acetylgruppe oder eine Benzoylgruppe bedeutet.

9. N-Phenylcarbamat, das Isopropyl - N - (3 - ethyl - 4 - ethoxyphenyl) - carbamat, Isopropyl - N - (3 - propyl - 4 - ethoxyphenyl) - carbamat, 1 - Methyl - 2 - propinyl - N - (3 - propyl - 4 - ethoxyphenyl) - carbamat, Isopropyl - N - (3 - methoxymethyl - 4 - ethoxyphenyl) - carbamat, sek. - Butyl - N - (3 - methoxymethyl - 4 - ethoxyphenyl) - carbamat, 1 - (Methoxymethyl) - ethyl - N - (3 - methoxymethyl - 4 - ethoxyphenyl) - carbamat oder Isopropyl - N - (3 - ethoxymethyl - 4 - ethoxyphenyl) - carbamat ist.

10. Verfahren zur Bekämpfung von pflanzenpathogenen Pilzen, dadurch gekennzeichnet, dass man eine fungizid wirksame Menge mindestens eines N-Phenylcarbamates der Formel: 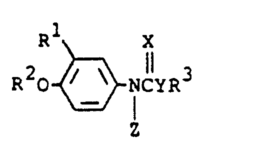 worin R¹, R², R³, X, Y und Z jeweils wie in Anspruch 1 definiert sind, auf pflanzenpathogene Pilze aufbringt.

11. Verfahren nach Anspruch 10, dadurch gekennzeichnet, dass die pflanzenpathogenen Pilze ein wirkstoffresistenter Stamm sind.

12. Verfahren zur Bekämpfung von pflanzenpathogenen Pilzen, dadurch gekennzeichnet, dass man eine fungizid wirksame Menge eines Gemisches eines N-Phenylcarbamates der Formel: 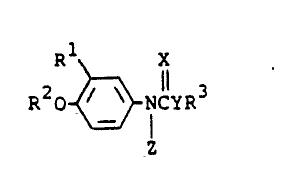 worin R¹, R², R³, X, Y und Z jeweils wie in Anspruch 1 definiert sind, und eines fungiziden Benzimidazols, fungiziden Thiophanants und/oder fungiziden cyclischen Imids auf pflanzenpathogene Pilze aufbringt.

13. Verfahren zur Herstellung eines N-Phenylcarbamates der Formel: 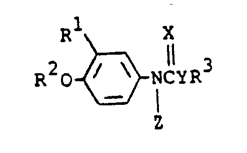 worin R', R², R³, X, Y und Z wie in Anspruch 6 definiert sind, dadurch gekennzeichnet, dass man ein Anilin der Formel: 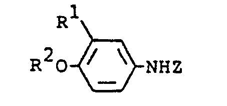 worin R¹, R² und Z jeweils wie oben definiert sind, mit einem Chlorformiat der Formel: 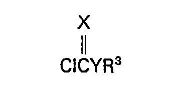 worin R³, X und Y jeweils wie oben definiert sind, umsetzt.

14. Verfahren zur Herstellung eines N-Phenylcarbamates der Formel: 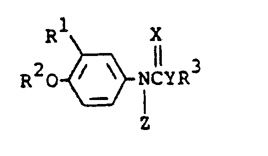 worin Z Wasserstoff bedeutet und R¹, R², R³, X und Y jeweils wie in Anspruch 6 definiert sind, dadurch gekennzeichnet, dass man ein Phenylisocyanat oder -isothiocyanat der Formel: 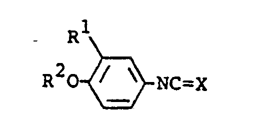 worin R¹, R² und X jeweils wie oben definiert sind, mit einem Alkohol oder Thiol der Formel: H_{YR3} worin R³ und Y jeweils wie oben definiert sind, umsetzt.

15. Verfahren zur Herstellung eines N-Phenylcarbamates der Formel: 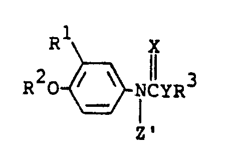 worin R¹, R², R³, X und Y jeweils wie in Anspruch 6 definiert sind und Z' eine niedere Alkylgruppe, eine - niedere Alkenylgruppe, eine niedere Alkinylgruppe, eine niedere Cycloalkylgruppe, eine niedere Halogenalkenylgruppe, eine niedere Alkylgruppe, die durch mindestens ein Halogenatom oder mindestens eine Cyano-, niedere Alkoxy- oder niedere Alkoxycarbonylgruppe substituiert ist, oder eine Gruppe der Formel: 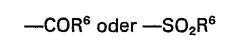 bedeutet, worin R⁶ eine niedere Alkylgruppe, eine niedere Alkenylgruppe, eine niedere Alkinylgruppe, eine niedere Cycloalkylgruppe, eine niedere Halogenalkenylgruppe, eiine niedere Alkylgruppe, die durch mindestens ein Halogenatom oder mindestens eine Cyano-, niedere Alkoxy, niedere Cycloalkyl- oder Phenoxygruppe substituiert ist (wobei die genannte Phenoxygruppe gegenbenenfalls durch mindestens ein Halogenatom und/oder mindestens eine Alkylgruppe substituiert ist), eine Phenylgruppe, eine Furylgruppe, eine Thienylgruppe, eine Phenylgruppe, die durch mindestens ein Halogenatom oder mindestens eine Cyano-, Nitro-, Trifluormethyl-, niedere Alkyl- oder niedere Alkoxygruppe substituiert ist, oder eine Aralkylgruppe bedeutet (wobei die genannte Aralkylgruppe gegebenenfalls durch mindestens ein Halogenatom und/oder mindestens eine Alkylgruppe substituiert ist), dadurch gekennzeichnet, dass man eine N-Phenylcarbamat der Formel: 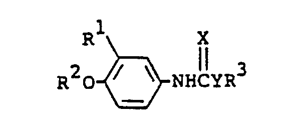 worin R¹, R², R³, X und Y jeweils wie oben definiert sind, mit einem Halogenid der Formel: 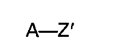 worin A ein Halogenatom bedeutet und Z' wie oben definiert ist, umsetzt.

## Patentansprüche (Patentansprüche für folgende(n) Vertragsstaat(en) : AT)

1. Verfahren zur Herstellung eines N-Phenylcarbamates der Formel: 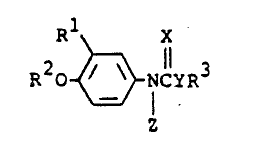 worin R¹ eine Alkylgruppe mit 2 bis 4 Kohlenstoffatomen, eine niedere Alkenylgruppe, eine niedere Cyanoalkenylgruppe, eine niedere Alkinylgruppe, eine niedere Alkylgruppe, die durch mindestens eine Hydroxyl- oder Cyanogruppe substituiert ist, oder eine Gruppe der Formel: 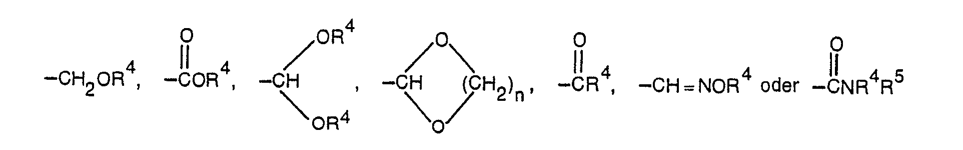 bedeutet, worin R⁴ eine niedere Alkylgruppe, eine niedere Alkenylgruppe, eine niedere Alkinylgruppe oder eine niedere Halogenalkylgruppe bedeutet, R⁵ ein Wasserstoffatom oder eine niedere Alkylgruppe bedeutet und n für 2, 3 oder 4 steht; R² eine niedere Alkylgruppe, eine niedere Alkenylgruppe, eine niedere Alkinylgruppe oder eine niedere Alkylgruppe, die durch mindestens ein Halogenatom oder mindestens eine niedere Alkoxy- oder niedere Cycloalkylgruppe substituiert ist, bedeutet; R³ eine Alkylgruppe mit 1 bis 8 Kohlenstoffatomen, eine Alkenylgruppe mit 3 bis 8 Kohlenstoffatomen, eine Alkinylgruppe mit 3 bis 8 Kohlenstoffatomen, eine niedere Cycloalkylgruppe, eine niedere Halogenalkenylgruppe, eine niedere Halogenalkinylgruppe, eine niedere Aralkylgruppe oder eine niedere Alkylgruppe, die durch mindestens ein Halogenatom oder mindestens eine Cyano-, niedere Alkoxy-, niedere Alkenyloxy-, niedere Halogenalkoxy, Phenoxy-, niedere Aralkyloxy- oder niedere Cycloalkylgruppe substituiert ist, bedeutet; X und Y jeweils unabhängig voneinander ein Sauerstoffatom oder ein Schwefelatom bedeuten; und Z ein Wasserstoffatom, eine niedere Alkylgruppe, eine niedere Alkenylgruppe, eine niedere Alkinylgruppe, eine niedere Cycloalkylgruppe, eine niedere Halogenalkenylgruppe, eine niedere Alkylgruppe, die durch mindestens ein Halogenatom oder mindestens eine Cyano-, niedere Alkoxy- oder niedere Alkoxycarbonylgruppe substituiert ist, oder eine Gruppe der Formel: 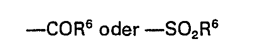 bedeutet, worin R⁶ eine niedere Alkylgruppe, eine niedere Alkenylgruppe, eine niedere Alkinylgruppe, eine niedere Cycloalkylgruppe, eine niedere Halogenalkenylgruppe, eine niedere Alkylgruppe, die durch mindestens ein Halogenatom oder mindestens eine Cyano-, niedere Alkoxy, niedere Cycloalkyl- oder Phenoxygruppe substituiert ist (wobei die Phenoxygruppe gegenbenenfalls durch mindestens ein Halogenatom und/oder mindestens eine Alkylgruppe substituiert ist), eine Phenylgruppe, eine Furylgruppe, eine Thienylgruppe, eine Phenylgruppe, die durch mindestens ein Halogenatom oder mindestens eine Cyano-, Nitro-, Trifluormethyl-, niedere Alkyl- oder niedere Alkoxygruppe substituiert ist, oder eine Aralkylgruppe bedeutet (wobei die genannte Aralkylgruppe gegebenenfalls durch mindestens ein Halogenatom und/oder mindestens eine Alkylgruppe substituiert ist), dadurch gekennzeichnet, dass man ein Anilin der Formel: 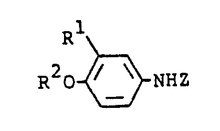 worin R¹, R² und Z jeweils wie oben definiert sind, mit einem Chlorformiat der Formel: 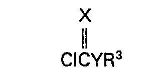 worin R³, X und Y jeweils wie oben definiert sind, umsetzt.

2. Verfahren zur Herstellung eines N-Phenylcarbamates der Formel: 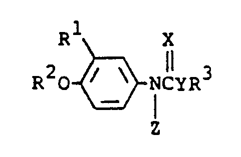 worin Z Wasserstoff bedeutet und R¹, R², R³, X und Y jeweils wie in Anspruch 1 definiert sind, dadurch gekennzeichnet, dass man ein Phenylisocyanat oder -isothiocyanat der Formel: 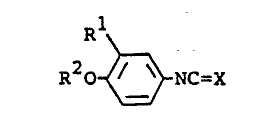 worin R¹, R² und X jeweils wie oben definiert sind, mit einem Alkohol oder Thiol der Formel: 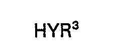 worin R³ und Y jeweils wie oben definiert sind, umsetzt.

3. Verfahren zur Herstellung eines N-Phenylcarbamates der Formel: 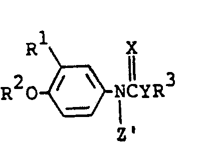 worin R¹, R², R³, X und Y jeweils wie in Anspruch 1 definiert sind und Z' eine niedere Alkylgruppe, eine niedere Alkenylgruppe, eine niedere Alkinylgruppe, eine niedere Cycloalkylgruppe, eine niedere Halogenalkenylgruppe, eine niedere Alkylgruppe, die durch mindestens ein Halogenatom oder mindestens eine Cyano-, niedere Alkoxy- oder niedere Alkoxycarbonylgruppe substituiert ist, oder eine Gruppe der Formel: 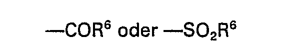 bedeutet, worin R⁶ eine niedere Alkylgruppe, eine niedere Alkenylgruppe, eine niedereAlkinylgruppe, eine niedere Cycloalkylgruppe, eine niedere Halogenalkenylgruppe, eine niedere Alkylgruppe, die durch mindestens ein Halogenatom oder mindestens eine Cyano-, niedere Alkoxy-, niedere Cycloalkyl- oder Phenoxygruppe substituiert ist (wobei die genannte Phenoxygruppe gegenbenenfalls durch mindestens ein Halogenatom und/oder mindestens eine Alkylgruppe substituiert ist), eine Phenylgruppe, eine Furylgruppe, eine Thienylgruppe, eine Phenylgruppe, die durch mindestens ein Halogenatom oder mindestens eine Cyano-, Nitro-, Trifluormethyl-, niedere Alkyl- und niedere Alkoxygruppe substituiert ist, oder eine Aralkylgruppe bedeutet (wobei die genannte Aralkylgruppe gegebenenfalls durch mindestens ein Halogenatom und/oder mindestens eine Alkylgruppe substituiert ist), dadurch gekennzeichnet, dass man ein N-Phenylcarbamat der Formel: 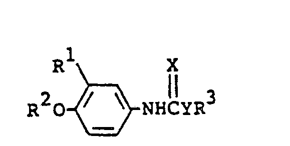 worin R¹, R², R³, X und Y jeweils wie oben definiert sind, mit einem Halogenid der Formel: 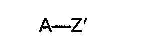 worin A ein Halogenatom bedeutet und Z' wie oben definiert ist, umsetzt.

4. Verfahren zur Bekämpfung von pflanzenpathogenen Pilzen, dadurch gekennzeichnet, dass man eine fungizid wirksame Menge mindestens eines der N-Phenylcarbamate der Formel: 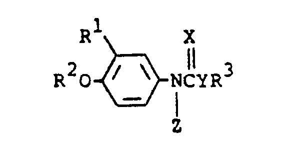 worin R' eine Alkylgruppe mit 2 bis 4 Kohlenstoffatomen, eine niedere Alkenylgruppe, eine niedere Cyanoalkenylgruppe, eine niedere Alkinylgruppe, eine niedere Alkylgruppe, die durch mindestens eine Hydroxyl- oder Cyanogruppe substituiert ist, oder eine Gruppe der Formel: 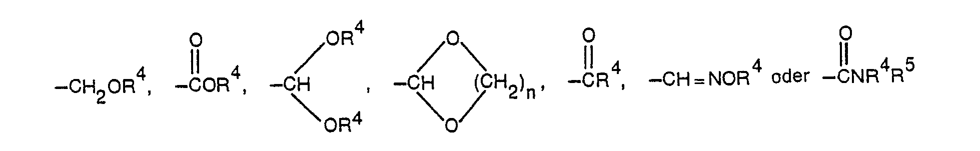 bedeutet, worin R⁴ eine niedere Alkylgruppe, eine niedere Alkenylgruppe, eine niedere Alkinylgruppe oder eine niedere Halogenalkylgruppe bedeutet, R⁵ ein Wasserstoffatom oder eine niedere Alkylgruppe bedeutet und n für 2, oder 4 steht; R² eine niedere Alkylgruppe, eine niedere Alkenylgruppe, eine niedere Alkinylgruppe oder eine niedere Alkylgruppe, die durch mindestens ein Halogenatom oder mindestens eine niedere Alkoxy- oder niedere Cycloalkylgruppe substituiert ist, bedeutet; R³ eine Alkylgruppe mit 1 bis 8 Kohlenstoffatomen, eine Alkenylgruppe mit 3 bis 8 Kohlenstoffatomen, eine Alkinylgruppe mit 3 bis 8 Kohlenstoffatomen, eine niedere Cycloalkylgruppe, eine niedere Halogenalkenylgruppe, eine niedere Halogenalkinylgruppe, eine niedere Aralkylgruppe oder eine niedere Alkylgruppe, die durch mindestens ein Halogenatom oder mindestens eine Cyano-, niedere Alkoxy-, niedere Alkenyloxy-, niedere Halogenalkoxy, Phenoxy-, niedere Aralkyloxy- oder niedere Cycloalkylgruppe substituiert ist, bedeutet; X und Y jeweils unabhängig voneinander ein Sauerstoffatom oder ein Schwefelatom bedeuten; und Z ein Wasserstoffatom, eine niedere Alkylgruppe, eine niedere Alkenylgruppe, eine niedere Alkinylgruppe, eine niedere Cycloalkylgruppe, eine niedere Halogenalkenylgruppe, eine niedere Alkylgruppe, die durch mindestens ein Halogenatom oder mindestens eine Cyano-, niedere Alkoxy- oder niedere Alkoxycarbonylgruppe-substituiert ist, oder eine Gruppe der Formel: 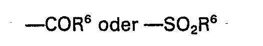 bedeutet, worin R⁶ eine niedere Alkylgruppe, eine niedere Alkenylgruppe, eine niedere Alkinylgruppe, eine niedere Cycloalkylgruppe, eine niedere Halogenalkenylgruppe, eine niedere Alkylgruppe, die durch mindestens ein Halogenatom oder mindestens eine Cyano-, niedere Alkoxy, niedere Cycloalkyl- oder Phenoxygruppe substituiert ist (wobei die genannte Phenoxygruppe gegenbenenfalls durch mindestens ein Halogenatom und/oder mindestens eine Alkylgruppe substituiert ist), eine Phenylgruppe, eine Furylgruppe, eine Thienylgruppe, eine Phenylgruppe, die durch mindestens ein Halogenatom oder mindestens eine Cyano-, Nitro-, Trifluormethyl-, niedere Alkyl- oder niedere Alkoxygruppe substituiert ist, oder eine Aralkylgruppe bedeutet (wobei die genannte Aralkylgruppe gegebenenfalls durch mindestens ein Halogenatom und/oder mindestens eine Alkylgruppe substituiert ist), auf pflanzenpathogene Pilze aufbringt.

5. Verfahren nach Anspruch 4, dadurch gekennzeichnet, dass die pflanzenpathogenen Pilze ein wirkstoffresistenter Stamm sind.

6. Verfahren zur Bekämpfung von pflanzenpathogenen Pilzen, dadurch gekennzeichnet, dass man eine fungizid wirksame Menge eines Gemisches eines N-Phenylcarbamates der Formel: 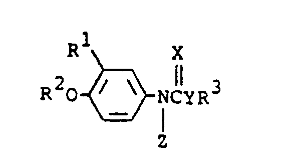 worin R¹, R², R³, X, Y und Z jeweils wie in Anspruch 1 definiert sind, und eines fungiziden Benzimidazols, fungiziden Thiophanats und/oder fungiziden cyclischen Imids aufbringt.

## Revendications (Revendications pour l'(les) Etat(s) contractant(s) suivant(s) : BE CH DE FR GB IT LI LU NL SE)

1. Composition fongicide, qui comprend comme substance active une quantité, efficace du point de vue fongicide, d'un N-phénylcarbamate de formule: 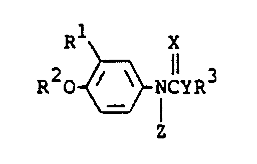 dans laquelle R¹ représente un groupe alkyle en C_{Z}-C₄, un groupe alcényle inférieur, un groupe cyano alcényle inférieur, un groupe alcynyle, un groupe alkyle inférieur substitué par au moins un groupe hydroxyle ou par un groupe cyano, ou un groupe de formule: 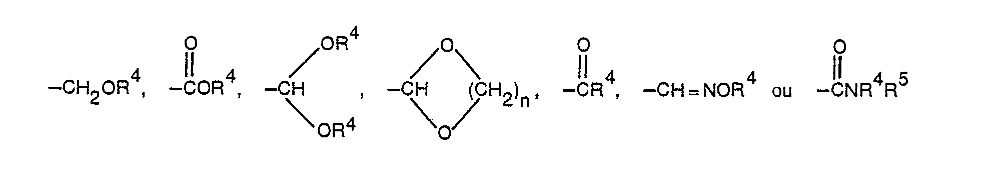 dans lesquelles R⁴ représente un groupe alkyle inférieur, un groupe alcényle inférieur, un groupe alcynyle inférieur ou un groupe halogénoalkyle inférieur, R⁵ représente un atome d'hydrogène ou un groupe alkyle inférieur et n vaut 2, 3 ou 4, R² représente un groupe alkyle inférieur, un groupe alcényle inférieur, un groupe alcynyle inférieur ou un groupe alkyle inférieur substitué par au moins un atome d'halogène, par un groupe alcoxy inférieur ou cycloalkyle inférieur; R³ représente un groupe alkyle en C₁―C₈, un groupe alcényle en C₃―C₈, un groupe alcynyle en C₃―C₈, un groupe cycloalkyle inférieur, un groupe halogénoalcényle inférieur, un groupe halogénoalcynyle inférieur, un groupe aralkyle inférieur ou un groupe alkyle inférieur (substitué par au moins un atome d'halogène, par un groupe cyano, alcoxy inférieur, alcényloxy inférieur, halogénoalcoxy inférieur, phénoxy, aralkyloxy inférieur ou cycloalkyle inférieur); X et Y représentent chacun, indépendamment, un atome d'oxygène ou un atome de soufre; et Z représente un atome d'hydrogène, un groupe alkyle inférieur, un groupe alcényle inférieur, un groupe alcynyle inférieur, un groupe cycloalkyle inférieur, un groupe halogénoalcényle inférieur, un groupe alkyle inférieur (substitué par au moins un atome d'halogène, un groupe cyano, alcoxy inférieur ou alcoxycarbonyle inférieur), ou un groupe de formule -COR⁶ ou ―SO₂R⁶, dans laquelle R⁶ représente un groupe alkyle inférieur, un groupe alcényle inférieur, un groupe alcynyle inférieur, un groupe cycloalkyle inférieur, un groupe alcynyle inférieur, un groupe cycloalkyle inférieur, un groupe halogénoalcényle inférieur, un groupe alkyle inférieur substitué par au moins un atome d'halogène, par un groupe cyano, alcoxy inférieur, cycloalkyl inférieur ou phénoxy (ledit groupe phénoxy étant éventuellement substitué par au moins un atome d'halogène et/ou au moins un groupe alkyle), un groupe phényle, un groupe furyle, un groupe thiényle, un groupe phényle substitué par au moins un atome d'halogène, par un groupe cyano, nitro, trifluorométhyle, alkyle inférieur ou alcoxy inférieur, ou un groupe aralkyle (ledit groupe aralkyle étant éventuellement substitué par au moins un atome d'halogène et/ou au moins un groue alkyle), avec un support, véhicule ou diluant inerte.

2. Composition fongicide selon la revendication 1, qui comprend en outre, à titre de substance active supplémentaire, au moins un fongicide de type benzimidazole, thiophanate ou imide cyclique.

3. Composition fongicide selon la revendication 2, dans laquelle le benzimidazole fongicide est le 1 - (butylcarbamoyl) - benzimidazole - 2 - yl - carbamate de méthyle, le 2 - (2 - furyl)benzimidazole, le 2 - (4 - thiazolyl)benzimidazole, ou le benzimidazole - 2 - yl - carbamate de méthyle.

4. Composition fongicide selon la revendication 2, dans laquelle le thiophanate fongicide est le 1,2 - bis(3 - méthoxycarbonyl - 2 - thiouréido)benzene, le 1,2 - bis(3 - éthoxycarbonyl - 2 - thiouréido)benzène, le 2 - (O,S - diméthylphosphorylamino) - 1 - (3' - méthoxycarbonyl - 2' - thiouréido)benzène ou le 2 - (O,0 - diméthylthiophosphorylamino) - 1 - (3' - méthoxycarbonyl - 2' - thiouréido)benzène.

5. Composition fongicide selon la revendication 2, dans laquelle l'imide cyclique fongicide est le 3 - (3',5' - dichlorophényl)-1,2 - diméthylcyclopropane - 1,2 - dicarboximide, la 3 - (3',5' dichlorophényl) - 1 - isopropylcarbamoylimidazolidine - 2,4 - dione, la 3 - (3',5' - dichlorophényl) - 5 - méthyl - 5 - vinyloxazolidine - 2,4 - dione ou le (RS) - -3(3',5' -dichlorophényl) 5 - méthyl - 2,4 - dioxooxazolidine - 5 - carboxylate d'éthyle.

6. N-phénylcarbamate de formule: 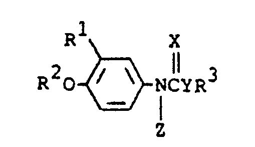 dans laquelle R¹ représente un groupe alkyle en C₂-C₄, un groupe alcényle inférieur, un groupe cyano alcényle inférieur, un groupe alcynyle inférieur, un groupe alkyle inférieur, substitué par au moins un groupe hydroxyle ou un groupe cyano, ou bien un groupe de formule: 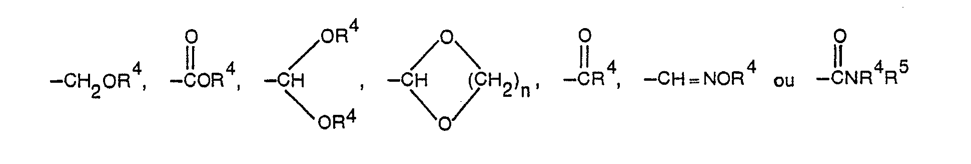 dans laquelle R⁴ représente un groupe alkyle inférieur, un groupe alcényle inférieur, un groupe alcynyle inférieur ou un groupe halogénoalkyle inférieur, R⁵ représente un atome d'hydrogène ou un groupe alkyle inférieur et n vaut 2, 3 ou 4; R² représente un groupe alkyle inférieur, un groupe alcényle inférieur, un groupe alcynyle inférieur ou un groupe alkyle inférieur (substitué par au moins un atome d'halogène, par un groupe alcoxy inférieur ou cycloalkyle inférieur); R³ représente un groupe alkyle en C₁-C₈, un groupe alcényle en C₃-Cg, un groupe alcynyle en C₃―C₈, un groupe cycloalkyle inférieur, un groupe halogénoalcényle inférieur, un groupe halogénoalcynyle inférieur, un groupe aralkyle inférieur, ou un groupe alkyle inférieur (substitué par au moins un atome d'halogène, par un groupe cyano, alcoxy inférieur, alcényloxy inférieur, halogénoalcoxy inférieur, phénoxy, aralkyloxy inférieur ou cycloalkyle inférieur); X et Y représentent chacun, indépendamment, un atome d'oxygène ou un atome de soufre; et Z représente un atome d'hydrogène, un groupe alkyle inférieur, un groupe alcényle inférieur, un groupe alcynyle inférieur, un groupe cycloalkyle inférieur, un groupe halogénoalcényle inférieur, un groupe alkyle inférieur (substitué par au moins un atome d'halogène, un groupe cyano, alcoxy inférieur ou alcoxycarbonyle inférieur), ou un groupe de formule: -COR⁶ ou -S0₂R', dans laquelle R⁶ repréente un groupe alkyle inférieur, un groupe alcényle inférieur, un groupe alcynyle inférieur, un groupe cycloalkyle inférieur, un groupe halogénoalcényle inférieur, un groupe alkyle inférieur (substitué par au moins un atome d'halogène, un groupe cyano, alcoxy inférieur, cycloalkyle inférieur ou phénoxy [ce groupe phénoxy étant éventuellement substitué par au moins un atome d'halogène et/ou au moins un groupe alkyle]), un groupe phényle, un groupe furyle, un groupe thiényle, un groupe phényle (substitué par au moins un atome d'halogène, un groupe cyano, nitro, trifluorométhyle, alkyle inférieur ou alcoxy inférieur), ou un groupe aralkyle (ledit groupe aralkyle étant éventuellement substitué par au moins un atome d'halogène et/ou au moins un groupe alkyle).

7. N-phénylcarbamate selon la revendication 6, dans lequel R¹ représente un groupe alkyle en C₂―C₄, un groupe alcényle en Cg-C₄, un groupe éthynyle, un groupe cyanométhyle, un groupe méthoxyméthyle, un groupe éthoxyméthyle, un groupe méthoxycarbonyle, un groupe éthoxycarbonyle, un groupe diméthoxyméthyle, un groupe méthylènedioxyméthyle, un groupe acétyle, un groupe propionyle, un groupe méthoxyiminométhyle ou un groupe N-éthylcarbamoyle; R² représente un groupe alkyle en Cₗ-C₃, un groupe allyle, un groupe propargyle, un groupe difluorométhyle, un groupe 2-chloréthyle, un groupe 2- fluoréthyle, un groupe 2,2,2-trifluoréthyle, un groupe 2-méthoxyéthyle ou un groupe 2-cyclopropyléthyle; R³ représente un groupe alkyle en C₁―C₈, un groupe alcényle en C₃-Ce, un groupe alcynyle en C₃―C₈, un groupe cyclobutyle, un groupe 4-chloro-2-butényle, un groupe 4-chloro-2-butynyle, un groupe 1- phényléthyle, un groupe 2-fluoréthyle, un groupe 1-(chlorométhyl)éthyle, un groupe 2-cyanoéthyle, un groupe 1-(méthoxyméthyl)éthyle, un groupe 2-chloro-1-(méthoxyméthyl)éthyle, un groupe 1-(allyloxyméthyl)éthyle, un groupe 1-(2-chloroéthoxyméthyl)éthyle, un groupe 1-(phénoxyméthyl)éthyle, un groupe 1-(benzyloxyméthyl)éthyle ou un groupe 1-(cyclopropyl)éthyle; X et Y représentent chacun, indépendamment, un atome d'oxygène ou un atome de soufre; et Z représente un atome d'hydrogène, un groupe méthyle, un groupe allyle, un groupe propargyle, un groupe cyanométhyle, un groupe éthoxycarbonylméthyle, un groupe acétyle, un groupe 1-méthylbutanoyle, un groupe cyclopropane- carbonyle, un groupe chloracétyle, un groupe benzoyle, un groupe 2-chlorobenzoyle ou un groupe méthylsulfonyle.

8. N-phénylcarbamate selon la revendication 6, dans lequel R¹ représente un groupe éthyle, un groupe propyle, un groupe allyle, un groupe méthoxyméthyle, un groupe éthoxyméthyle, ou un groupe éthoxycarbonyle; R² représente un groupe éthyle, un groupe propyle, un groupe allyle, un groupe propargyle ou un groupe 2-chloréthyle; R³ représente un groupe méthyle, un groupe éthyle, un groupe isopropyle, un groupe butyle secondaire, un groupe 1-méthyl-2-propényle, un groupe 1-méthyle-2-propynyle, un groupe 4-chloro-2-butynyle, un groupe 1-phényléthyle, un groupe 2-fluoréthyle, un groupe 1-(chlorométhyl)éthyle, un groupe 2-cyanoéthyle ou un groupe 1-(méthoxyméthyl)éthyle; X représente un atome d'hydrogène; Y représente un atome d'hydrogène ou un atome de soufre; et Z représente un atome d'hydrogène, un groupe acétyle ou un groupe benzoyle.

9. N-phénylcarbamate qui est le N - (3 - éthyl - 4 - éthoxyphényl)carbamate d'isopropyle, le N - (3 - propyl - 4 - éthoxyphényl) - carbamate d'isopropyle, le N - (3 - propyl - 4 - éthoxyphényl)carbamate de 1 - méthyl - 2 - propynyle, le N - (3 - méthoxyméthyl - 4 - éthoxyphényl)carbamate d'isopropyle, le N - (3 - méthoxyméthyl - 4 - éthoxyphényl)carbamate de butyle secondaire, le N - (3 - méthoxyméthyl - 4 - éthoxyphényl)carbamate de 1 - (méthoxymethyl)éthyle ou le N - (3 - éthoxyméthyl - 4 - éthoxyphényl)carbamate d'isopropyle.

10. Procédé pour maîtriser des champignons phytopathogènes qui comprend l'application d'une quantité, efficace du point de vue fongicide, d'au moins un N-phénylcarbamate de formule: 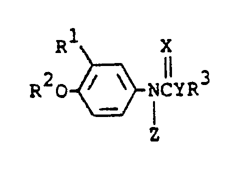 dans laquelle R¹, R², R³, X, Y et Z sont chacun tels que définis à la revendication 1, sur des champignons phytopathogènes.

11. Procédé selon la revendication 10, dans lequel les champignons phytopathogènes constituent une souche résistante aux médicaments ou produits chimiques.

12. Procédé pour maîtriser des champignons phytopathogènes, qui comprend l'application, aux champignons phytopathogènes, d'une quantité, efficace du point de vue fongicide, d'un mélange d'un N-phénylcarbamate de formule: 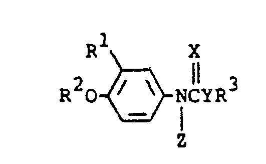 dans laquelle R¹, R², R³, X, Y et Z sont chacun tels que définis à la revendication 1, et d'un fongicide de type benzimidazole, thiophanate et/ou imide cyclique.

13. Procédé pour produire un N-phénylcarbamate de formule: 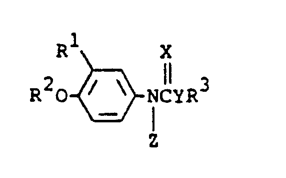 dans laquelle R¹, R², R³, X, Y et Z sont tels que définis à la revendication 1, ce procédé comprenant la réaction d'une aniline de formule: 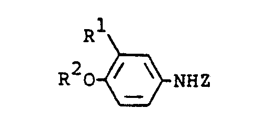 dans laquelle R¹, R² et Z sont tels que définis ci-dessus, avec un chloroformiate de formule: 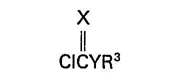 dans laquelle R³, X et Y sont tels que définis ci-dessus.

14. Procédé pour produire un N-phénylcarbamate de formule: 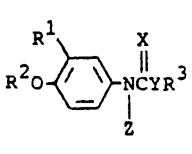 dans laquelle Z représente un atome d'hydrogène et R¹, R², R³, X et Y sont chacun tels que définis revendication 6, ce procédé comprenant la réaction d'un isocyanate ou isothiocyanate de phény formule: 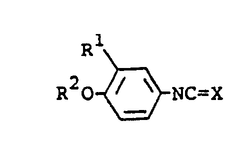 dans laquelle R¹, R² et X sont chacun tels que définis ci-dessus, avec un alcool ou thiol de formule: 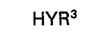 dans laquelle R³ et Y sont chacun tels que définis ci-dessus.

15. Procédé pour produire un N-phénylcarbamate de formule: 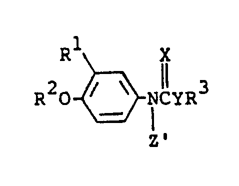 dans laquelle R¹, R², R³, X et Y sont chacun tels que définis à la revendication 6 et Z' représente un gn alkyle inférieur, un groupe alcényle inférieur, un groupe alcynyle inférieur, un groupe cycloalkyle infé un groupe halogénoalcényle inférieur, un groupe alkyle inférieur (substitué par au moins un a1 d'halogène, un groupe cyano, alcoxy inférieur ou alcoxycarbonyle inférieur), ou un groupe de fon ―COR⁶ ou ―SO₂R⁶, dans lequels R⁶ représente un groupe alkyle inférieur, un groupe alcényle inférieu groupe alcynyle inférieur, un groupe cycloalkyle inférieur, un groupe halogénoalcényle inférieui groupe alkyle inférieur (substitué par au moins un atome d'halogène, un groupe cyano, alcoxy infé cycloalkyl inférieur ou phénoxy [ledit groupe phénoxy étant éventuellement substitué par au moir atome d'halogène et/ou au moins un groupe alkyle]), un groupe phényle, un groupe furyle, un gr thiényle, un groupe phényle (substitué par au moins un atome d'halogène, un groupe cyano, i trifluorométhyle, alkyle inférieur ou alcoxy inférieur [ou un groupe aralkyle, ledit groupe aralkyle éventuellement substitué par au moins un atome d'halogène et/ou au moins un groupe alkyle]) comprend la réaction d'un N-phénylcarbamate de formule: 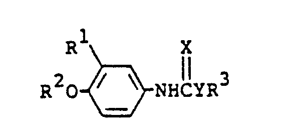 dans laquelle R¹, R², R³, X et Y sont chacun tels que définis ci-dessus, avec un halogénure de form 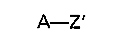 dans laquelle A représente un atome d'halogène et Z' est tel que défini ci-dessus.

## Revendications (Revendications pour l'(les) Etat(s) contractant(s) suivant(s) : AT)

1. Procédé pour produire un N-phénylcarbamate de formule: 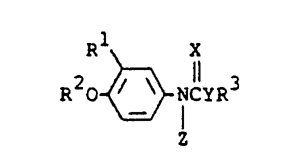 dans laquelle R¹ représente un groupe alkyle en C₂―C₄, un groupe alcényle inférieur, un groupe cyano alcényle inférieur, un groupe alcynyle inférieur, un groupe alkyle inférieur, substitué par au moins un groupe hydroxyle ou cyano, ou bien un groupe de formule: 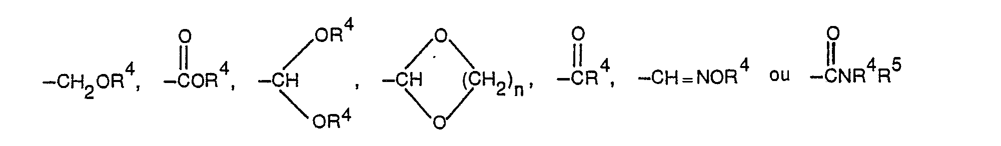 dans lesquelles R⁴ représente un groupe alkyle inférieur un groupe alcényle inférieur un groupe alcynyle inferieur, ou un groupe halogénoalkyle inférieur, R⁵ représente un atome d'hydrogène ou un groupe alkyle inférieur et n vaut 2, 3 ou 4; R² représente un groupe alkyle inférieur, un groupe alcényle inférieur, un groupe alcynyle inférieur ou un groupe alkyle inférieur substitué, par au moins un atome d'halogène, un groupe alcoxy inférieur ou cycloalkyle inférieur; R³ représente un groupe alkyle en C₁―C₈, un groupe alcényle en C₃―C₈, un groupe alcynyle en C₃-Cg, un groupe cycloalkyle inférieur, un groupe halogénoalcényle inférieur, un groupe halogénoalcynyle inférieur, un groupe aralkyle inférieur ou un groupe alkyle inférieur (substitué par au moins un atome d'halogène, un groupe cyano, alcoxy inférieur, alcényloxy inférieur, halogénoalcoxy inférieur, phénoxy, aralkyloxy inférieur ou cycloalkyle inférieur); X et Y représentent chacun, indépendamment, un atome d'oxygène ou un atome de soufre; et Z représente un atome d'hydrogène, un groupe alkyle inférieur, un groupe alcényle inférieur, un groupe alcynyle inférieur, un groupe cycloalkyle inférieur, un groupe halogénoalcényle inférieur, un groupe alkyle inférieur (substitué par au moins un atome d'halogène, un groupe cyano, alcoxy inférieur ou alcoxycarbonyle inférieur), ou un groupe de formule: -COR⁶ ou ―SO₂R₆, dans laquelle R⁶ représente un groupe alkyle inférieur, un groupe alcényle inférieur, un groupe alcynyle inférieur, un groupe cycloalkyle inférieur, un groupe halogénoalcényle inférieur, un groupe alkyle inférieur (substitué par au moins un atome d'halogène, un groupe cyano, alcoxy inférieur, cycloalkyle inférieur ou phénoxy [le groupe phénoxy étant éventuellement substitué par au moins un atome d'halogène et/ou au moins un groupe alkyle]), un groupe phényle, un groupe furyle, un groupe thiényle, un groupe phényle (substitué par au moins un atome_{;}d'halogène, par un groupe cyano, nitro, trifluorométhyle, alkyle inférieur ou alcoxy inférieur) ou un groupe aralkyle (ledit groupe aralkyle étant éventuellement substitué par au moins un atome d'halogène et/ou au moins un groupe alkyle), qui comprend la réaction d'une aniline de formule: 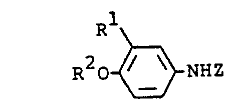 dans laquelle R¹, R² et Z sont chacun tels que définis ci-dessus, avec un chloroformiate de formule: 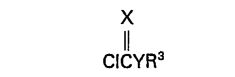 dans laquelle R', X et Y sont chacun tels que définis ci-dessus.

2. Procédé pour produire un N-phénylcarbamate de formule: 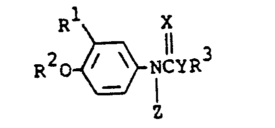 dans laquelle Z représente un atome d'hydrogène et R¹, R², R³, X et Y sont chacun tels que définis à la revendication 1, qui comprend la réaction d'un isocyanate ou isothiocyanate de phényle de formule: 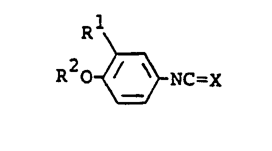 dans laquelle R¹, R² et X sont chacun tels que définis ci-dessus, avec un alcool ou thiol de formule: 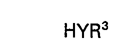 dans laquelle R³ et Y sont chacun tels que définis ci-dessus.

3. Procédé pour produire un N-phénylcarbamate de formule: 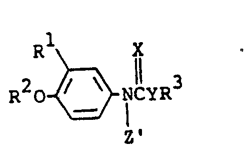 dans laquelle R¹, R², R³, X et Y sont chacun tels que définis à la revendication 1 et Z' représente un groupe alkyle inférieur, un groupe alcényle inférieur, un groupe alcynyle inférieur, un groupe cycloalkyle inférieur, un groupe halogénoalcényle inférieur, un groupe alkyle inférieur (substitué par au moins un atome d'halogène, un groupe cyano, alcoxy inférieur ou alcoxycarbonyle inférieur), ou un groupe de formule: -COR⁶ ou ―SO₂R⁶, dans lesquelles R⁶ représente un groupe alkyle inférieur, un groupe alcényle inférieur, un groupe alcynyle inférieur, un groupe cycloalkyle inférieur, un groupe halogénoalcényle inférieur, un groupe alkyle inférieur (substitué par au moins un atome d'halogène, un groupe cyano, alcoxy inférieur, cycloalkyl inférieur ou phénoxy [ledit groupe phénoxy étant éventuellement substitué par au moins un atome d'halogène et/ou au moins un groupe alkyle]), un groupe phényle, un groupe furyle, un groupe thiényle, un groupe phényle (substitué par au moins un atome d'halogène, par un groupe cyano, nitro, trifluorométhyle, alkyle inférieur ou alcoxy inférieur) ou un groupe aralkyle ledit groupe aralkyle étant éventuellement substitué par au moins un atome d'halogène et/ou au moins un groupe alkyle), ce procédé comprenant la réaction d'un N-phénylcarbamate de formule: 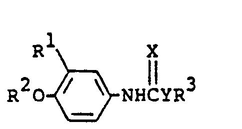 dans laquelle R¹, R², R³, X et Y sont chacun tels que définis ci-dessus, avec un halogénure de formule: 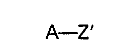 dans laquelle A représente un atome d'halogène et Z' est tel que défini ci-dessus.

4. Procédé pour maîtriser des champignons phytopathogènes, qui comprend l'application aux champignons phytopathogènes d'une quantité, efficace du point de vue fongicide, d'au moins un N-phénylcarbamate de formule: 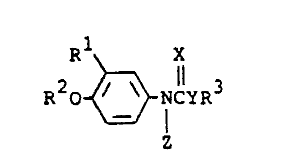 dans laquelle R¹ représente un groupe alkyle en C₂₋C₄, un groupe alcényle inférieur, un groupe cyanoalcényle inférieur, un groupe alcynyle inférieur, un groupe alkyle inférieur (substitué par au moins un groupe hydroxyle ou cyano) ou un groupe de formule: 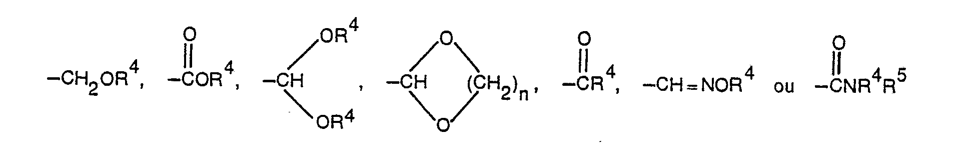 dans lesquelles R⁴ représente un groupe alkyle inférieur, un groupe alcényle inférieur, un groupe alcynyle inférieur ou un groupe halogénoalkyle inférieur, R⁵ représente un atome d'hydrogène ou un groupe alkyle inférieur et n vaut 2, 3 ou 4; R² représente un groupe alkyle inférieur un groupe alcényle inférieur, un groupe alcynyle inférieur ou un groupe alkyle inférieur substitué, par au moins un atome d'halogène, un groupe alcoxy inférieur ou cycloalkyle inférieur; R³ représente un groupe alkyle en C₁―C₈, un groupe alcényle en C₃―C₈, un groupe alcynyle en C₃―C₈, un groupe cycloalkyle inférieur, un groupe halogénoalcényle inférieur, un groupe halogénoalcynyle inférieur, un groupe aralkyle inférieur ou un groupe alkyle inférieur (substitué par au moins un atome d'halogène, un groupe cyano, alcoxy inférieur, alcényloxy inférieur, halogénoalcoxy inférieur, phénoxy, aralkyloxy inférieur ou cycloalkyle inférieur); X et Y représentent chacun, indépendamment, un atome d'oxygène ou un atome de soufre; et Z représente un atome d'hydrogène, un groupe alkyle inférieur, un groupe alcényle inférieur, un groupe alcynyle inférieur, un groupe cycloalkyle inférieur, un groupe halogénoalcényle inférieur, un groupe alkyle inférieur (substitué par au moins un atome d'halogène, un groupe cyano, alcoxy inférieur ou alcoxycarbonyle inférieur), ou un groupe de formule: -COR⁶ ou -SO_{Z}R⁶, dans laquelle R⁶ représente un groupe alkyle inférieur, un groupe alcényle inférieur, un groupe alcynyle inférieur, un groupe cycloalkyle inférieur, un groupe halogénoalcényle inférieur, un groupe alkyle inférieur (substitué par au moins un atome d'halogène, un groupe cyano, alcoxy inférieur, cycloalkyle inférieur ou phénoxy [ledit groupe phénoxy étant éventuellement substitué par au moins un atome d'halogène et/ou au moins un groupe alkyle]), un groupe phényle, un groupe furyle, un groupe thiényle, un groupe phényle (substitué par au moins un atome d'halogène, par un groupe cyano, nitro, trifluorométhyle, alkyle inférieur ou alcoxy inférieur), ou un groupe aralkyle (ledit groupe aralkyle étant éventuellement substitué par au moins un atome d'halogène et/ou au moins un groupe alkyle).

5. Procédé selon la revendication 4, dans lequel les champignons phytopathogènes constituent une souche résistante aux médicaments ou aux produits chimiques.

6. Procédé pour maîtriser des champignons phytopathogènes qui comprend l'application d'une quantité, efficace du point de vue fongicide, d'un mélange d'un N-phénylcarbamate de formule: 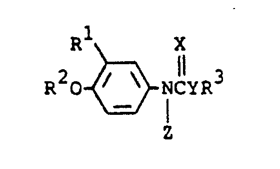 dans laquelle R¹, R², R³, X, Y et Z sont chacun tels que définis à la revendication 1, et d'un fongicide de type benzimidazole, thiophanate et/ou imide cyclique.
